# EUROPEAN PATENT APPLICATION

(11) **EP 1 266 664 A1**
(43) Date of publication of application: **18.12.2002**
(21) Application number: 01908257.7
(22) Date of filing: 02.03.2001
(51) Int. Cl.: A61K 45/00, A61K 31/404, A61K 31/4439, A61K 31/496, A61K 31/405

(54) **PREPARATIONS FOR CHONDROGENESIS**

(30) Priority: 10.03.2000 JP 2000067499
(71) Applicant: CHUGAI SEIYAKU KABUSHIKI KAISHA, Tokyo, 115-8543 (JP)
(72) Inventor: NAGAO, Shunsuke, Chugai Seiyaku Kabushiki Kaisha, Shizuoka 412-8513 (JP); KITAMURA, Hidetomo, Chugai Seiyaku KK, Gotenba-shi, Shizuoka 412-8513 (JP); HAYASHI, Yoshiki, Chugai Seiyaku Kabushiki Kaisha, Gotenba-shi, Shizuoka 412-8513 (JP)
(74) Representative: HOFFMANN - EITLE
(86) International application number: JP0101630
(87) International publication number: WO01066142

(57) **Abstract**

A chondrogenic composition comprising a drug with chondrogenic effect, a biodegradable and/or bioerodable polymer and a water-soluble and/or water-dispersible organic solvent.

## Description

### Technical Field

The present invention relates to a chondrogenic composition.

### Background Art

Human cartilage is considered to be unregenerable in the event of loss of a region of 3 mm or more. Damage produced in cartilage is therefore treated by transplantation of autologous chondrocytes. Methods of repairing damaged cartilage by artificial joints have also been implemented.

At the same time, research is also progressing on drugs with chondrogenic effects. Research is also being conducted in the field of regenerative medical engineering (tissue engineering), whereby porous matrices composed of biodegradable polymers such as collagen or polylactic acid are embedded at sites of cartilage damage.

### Disclosure of the Invention

However, repair of damaged cartilage by transplantation of autologous chondrocytes requires a high level of skill and sophisticated equipment for surgery, while the use of artificial joints is also associated with problems in terms of biocompatibility and durability.

Although biologically derived growth factors have been reported as drugs with chondrogenic effects, there has not yet been developed a useful formulation suitable for administration to patients in the clinic. Moreover, using porous matrices composed of biodegradable polymers such as collagen or polylactic acid has not provided satisfactory repair and regeneration of cartilage.

A composition which provides an implant for tissue regeneration has been disclosed in Japanese Patent Publication No. 2992046, but there is no mention in this publication regarding repair, regeneration or formation of cartilage tissue.

It is an object of the present invention, which has been accomplished in light of these technical problems, to provide a chondrogenic composition which does not require a high level of skill or sophisticated equipment for treatment, and which offers excellent biocompatibility as well as adequately high efficiency for repair and regeneration of cartilage.

The present inventors conducted persistent research with the aim of achieving the object stated above, based on the knowledge that efficient repair and regeneration of damaged cartilage in the body requires the presence of a drug with chondrogenic effect, a scaffold to which the chondrocytes can attach and a space in which the chondrocytes can proliferate and form an extracellular matrix, and as a result we have completed the present invention upon finding that said object can be achieved by a formulation comprising a drug with chondrogenic effect, a biocompatible polymer and an organic solvent.

In other words, the present invention is characterized by comprising a drug with chondrogenic effect, a biodegradable and/or bioerodable polymer and a water-soluble and/or water-dispersible organic solvent.

According to the invention, the aforementioned drug with chondrogenic effect is preferably a compound represented by the following general formula (I) or a salt thereof. 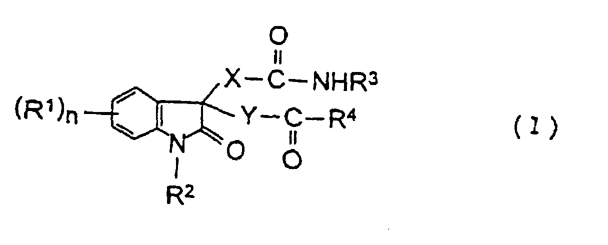 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent;
R³ represents a lower alkyl group with an optional substituent, a cycloalkyl group with an optional substituent, an aryl group with an optional substituent or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group;
X and Y may be the same or different and each represents -CH₂-, -NH- or -O-, and n represents an integer of 0-4.

According to the invention, preferably R² in general formula (I) above is a 2,2-diethoxyethyl group, R³ is a 4-methylphenyl group, R⁴ is -NHR⁷ wherein R⁷ is a 4-methylphenyl group, X and Y are the same or different and each is -CH₂- or -NH-, and n is 0.

According to the invention, the aforementioned drug with chondrogenic effect is preferably a compound represented by the following general formula (IV) or a salt thereof. 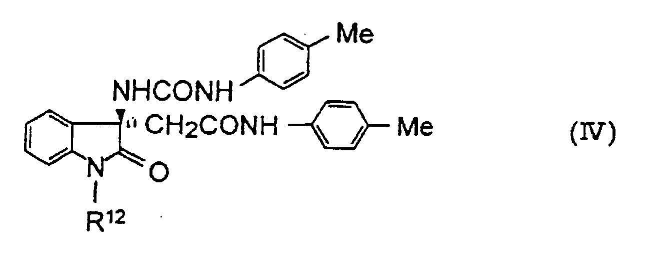 wherein R¹² represents a lower alkyl group substituted at the same carbon with two lower alkoxy groups which is substituted with 1-5 halogen atoms.

Preferably, R¹² in general formula (IV) above is a group represented by general formula (V): 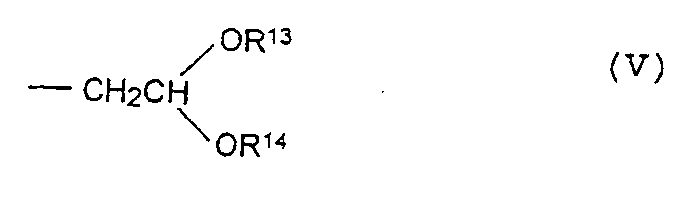 wherein R¹³ and R¹⁴ may be the same or different, and each represents a lower alkyl group substituted with 1-5 halogen atoms.

According to the invention, the aforementioned polymer is preferably a porous body-forming polymer which holds proliferated chondrocytes induced by said drug with chondrogenic effect in the body, and the polymer also preferably has a weight-average molecular weight of 500 or greater.

The polymer is preferably at least one polymer selected from the group consisting of polyhydroxyalkanoic acids, lactone ring-opened polymers, hydroxyalkanoic acid/lactone copolymers, polyacetals, polyketals, polymethylvinyl ether, chitin, chitosan, polyamides, polyamino acids, polyurethane, polyester amides, polycarboxylic acids, polyanhydrides, polyalkylenes, polyphosphoric acid, polyortho esters and copolymers containing at least one of these in the molecule.

The invention further provides a chondrogenic composition wherein at least a portion of the polymer is a reactive polymer having an unsaturated double bond.

According to the invention, the reactive polymer is preferably a compound having a skeleton comprising at least one polymer selected from the group consisting of polyhydroxyalkanoic acids, lactone ring-opened polymers, hydroxyalkanoic acid/lactone copolymer, polyacetals, polyketals, polymethylvinyl ether, chitin, chitosan, polyamides, polyamino acids, polyurethane, polyester amides, polycarboxylic acids, polyanhydrides, polyalkylenes, polyphosphoric acid, polyortho esters and copolymers containing at least one of these in the molecule, with at least one (meth)acryloyl group bonded to the skeleton.

According to the invention, the organic solvent is preferably at least one organic solvent selected from the group consisting of N-methyl-2-pyrrolidone, 2-pyrrolidone, ethanol, benzyl alcohol, propyleneglycol, acetone, ethyl acetate, methyl ethyl ketone, dimethylformamide, dimethylsulfoxide, tetrahydrofuran, caprolactam, N,N-dimethylacetamide, triacetin, glycerol, polyoxyethylene-hardened castor oil, vitelline phospholipid, soy lecithin, polyoxyethylene sorbitan fatty ester and polyoxyethylene alkylether.

The invention further provides a chondrogenic composition which comprises a drug with chondrogenic effect, a biodegradable and/or bioerodable polymer, a water-soluble and/or water-dispersible organic solvent, and a water-soluble compound.

The water-soluble compound preferably has a mean particle size of 1-3000 µm, and the water-soluble compound is preferably at least one water-soluble compound selected from the group consisting of monosodium phosphate, monosodium phosphate hydrate, disodium phosphate, disodium phosphate hydrate, sodium chloride, magnesium sulfate, magnesium sulfate hydrate, magnesium sulfate double salts, magnesium hydroxide, glucose, mannitol, xylose, sucrose, fructose, hyaluronic acid, dextran, amino acids and polypeptides. The water-soluble compound is also preferably a compound with a pH of 6 or higher in aqueous solution.

According to the invention, preferably the content of the drug with chondrogenic effect is 0.0001-50 wt%, the content of the polymer is 5-80 wt% and the content of the organic solvent is 5-90 wt%, and preferably the content of the water-soluble compound is no greater than 90 wt%.

### BRIEF DESCRIPTION OF THE DRAWINGS

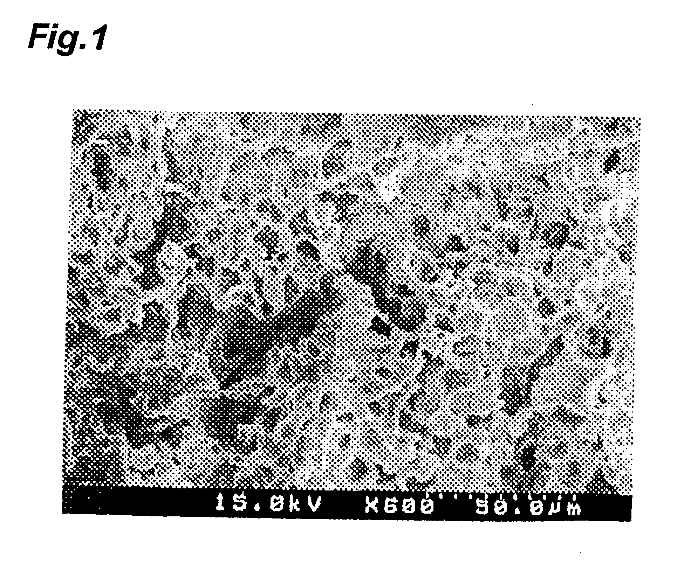

Fig. 1 is a scanning electron microscope photograph of precipitated (insolubilized) PLA0005 obtained by immersing a chondrogenic composition containing PLA0005, compound A and NMP in purified water and incubating at 37°C for 3 days to solidification.

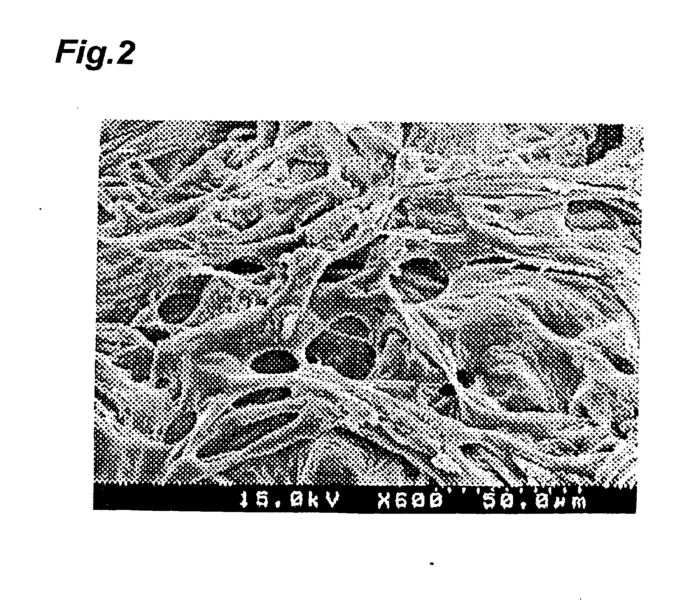

Fig. 2 is a scanning electron microscope photograph of precipitated (insolubilized) PLGA5020 obtained by immersing a chondrogenic composition containing PLGA5020, compound A and NMP in purified water and incubating at 37°C for 3 days to solidification.

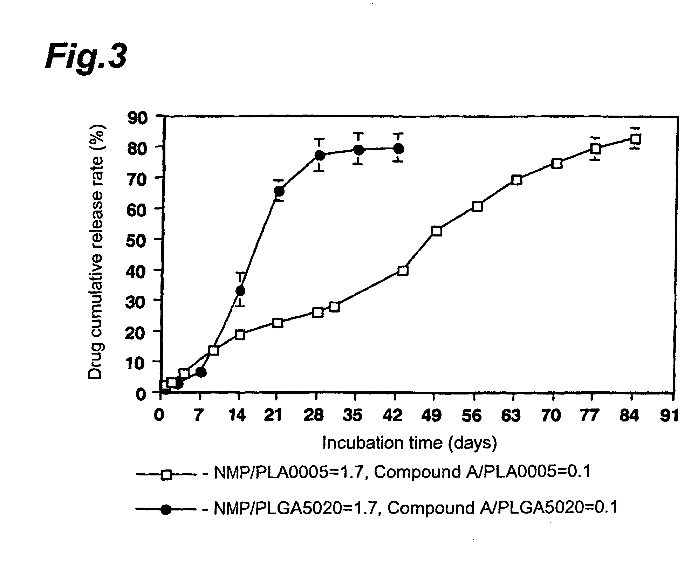

Fig. 3 is a graph showing the relationship between the incubation times for a chondrogenic composition containing PLA0005, compound A and NMP and a chondrogenic composition containing PLGA5020, compound A and NMP, and the cumulative release rates for compound A.

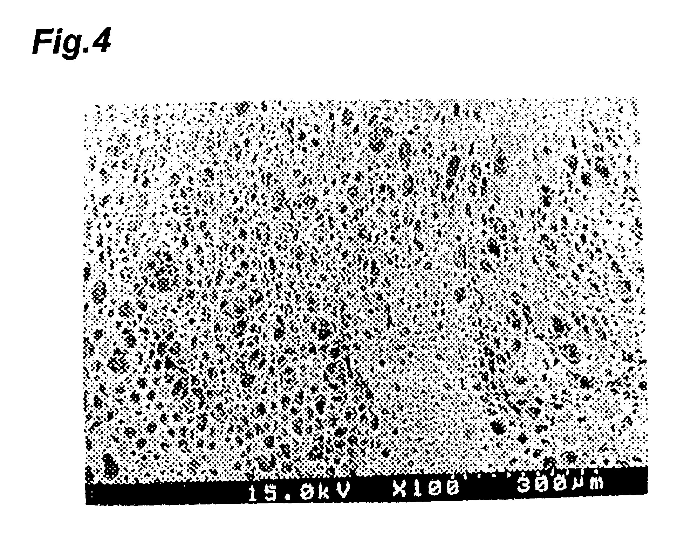

Fig. 4 is a scanning electron microscope photograph (100x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.2) in purified water and incubating at 37°C for 3 days to solidification.

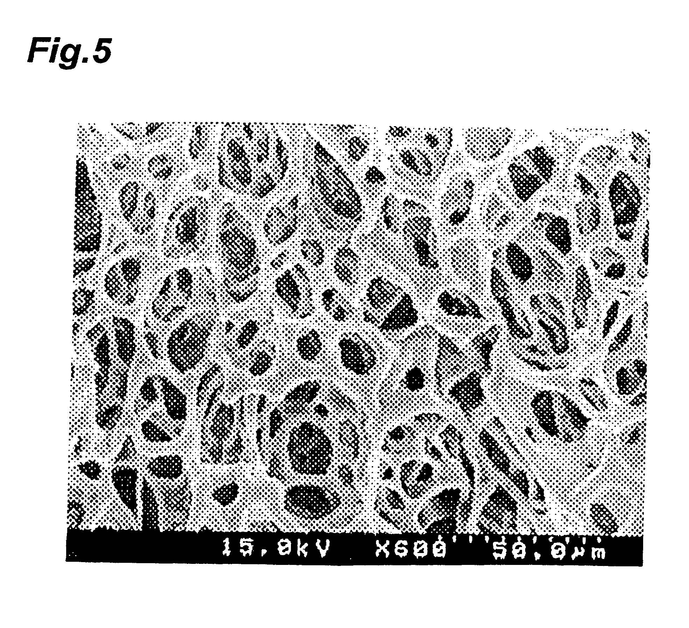

Fig. 5 is a scanning electron microscope photograph (600x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.2) in purified water and incubating at 37°C for 3 days to solidification.

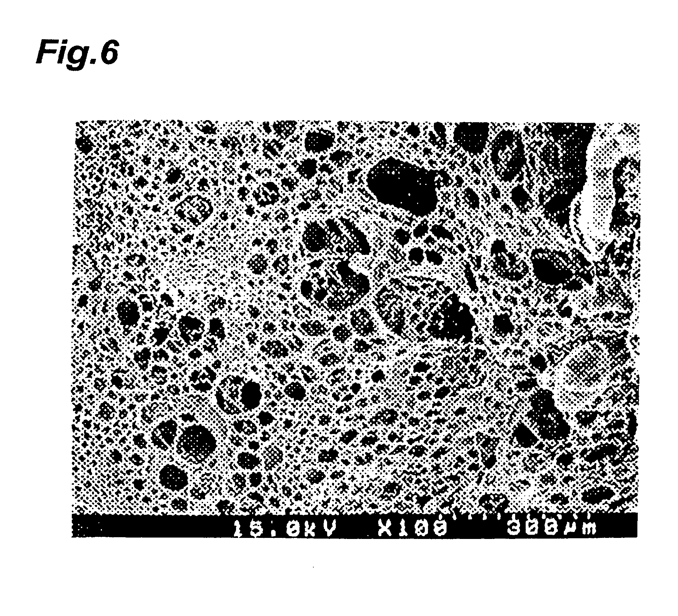

Fig. 6 is a scanning electron microscope photograph (100x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.3) in purified water and incubating at 37°C for 3 days to solidification.

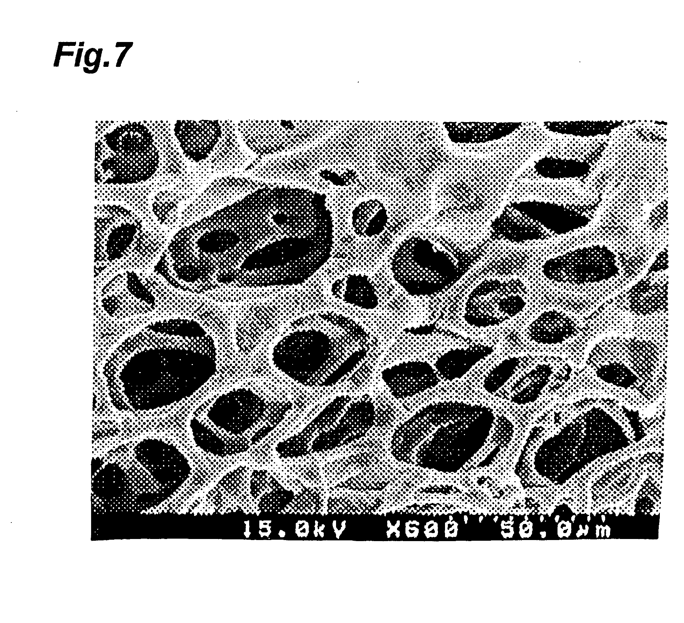

Fig. 7 is a scanning electron microscope photograph (600x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.3) in purified water and incubating at 37°C for 3 days to solidification.

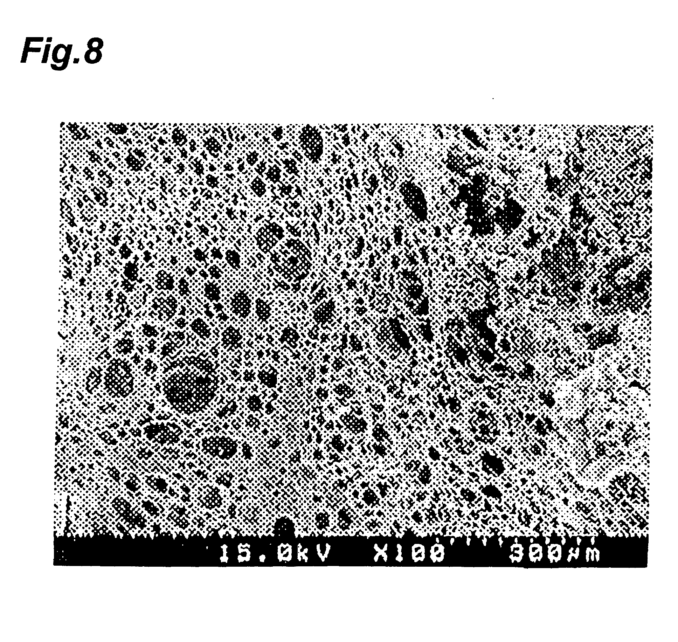

Fig. 8 is a scanning electron microscope photograph (100x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.4) in purified water and incubating at 37°C for 3 days to solidification.

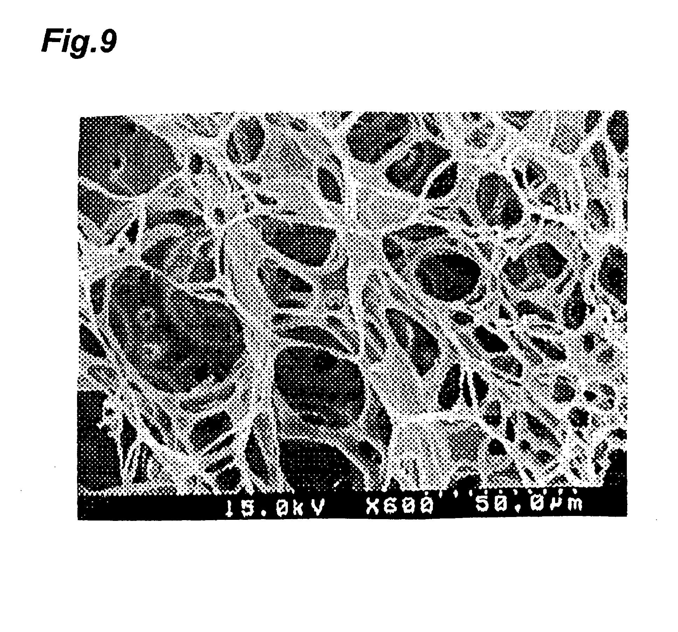

Fig. 9 is a scanning electron microscope photograph (600x magnification) of precipitated (insolubilized) PLGA5010 obtained by immersing a drug-free formulation containing PLGA5010, NMP and disodium phosphate dodecahydrate (disodium phosphate dodecahydrate/PLGA5010 = 0.4) in purified water and incubating at 37°C for 3 days to solidification.

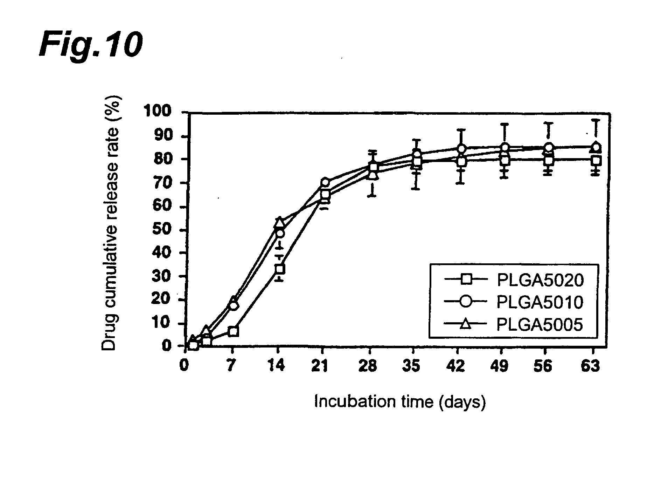

Fig. 10 is a graph showing the relationship between the incubation times for a chondrogenic composition containing PLGA5020, compound A and NMP, a chondrogenic composition containing PLGA5010, compound A and NMP and a chondrogenic composition containing PLGA5005, compound A and NMP, and the cumulative release rates for compound A.

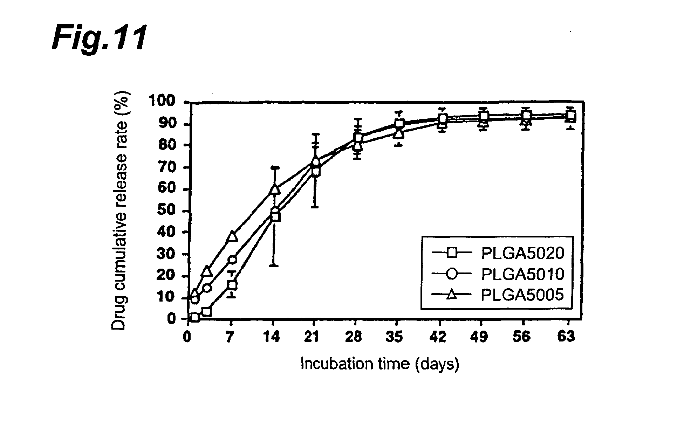

Fig. 11 is a graph showing the relationship between the incubation times for a chondrogenic composition containing PLGA5020, compound A, NMP and disodium phosphate dodecahydrate, a chondrogenic composition containing PLGA5010, compound A, NMP and disodium phosphate dodecahydrate and a chondrogenic composition containing PLGA5005, compound A, NMP and disodium phosphate dodecahydrate, and the cumulative release rates for compound A.

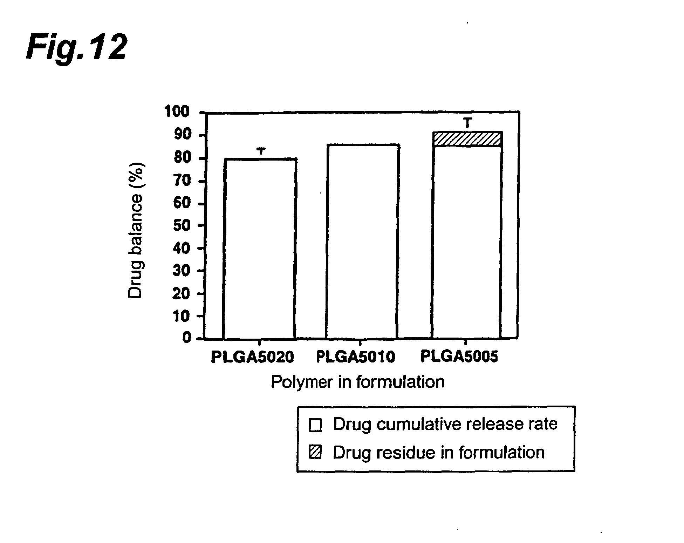

Fig. 12 is a graph showing the drug balance for a chondrogenic composition containing PLGA5020, compound A and NMP, a chondrogenic composition containing PLGA5010, compound A and NMP and a chondrogenic composition containing PLGA5005, compound A and NMP.

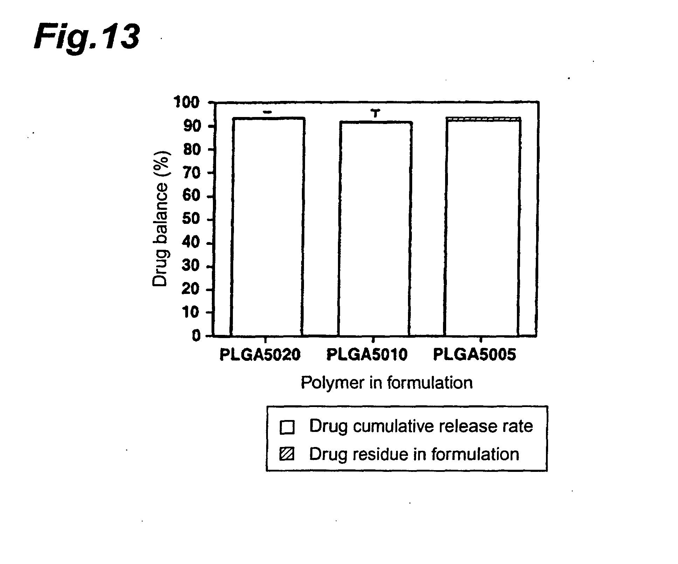

Fig. 13 is a graph showing the drug balance for a chondrogenic composition containing PLGA5020, compound A, NMP and disodium phosphate dodecahydrate, a chondrogenic composition containing PLGA5010, compound A, NMP and disodium phosphate dodecahydrate and a chondrogenic composition containing PLGA5005, compound A, NMP and disodium phosphate dodecahydrate.

### Best Mode for Carrying Out the Invention

The invention will now be explained in further detail with reference to the attached drawings as necessary. The "parts" and "%" values indicating the dose ratios in the description which follows are based on weight, unless otherwise specified.

The chondrogenic composition of the invention comprises a drug with chondrogenic effect, a biodegradable and/or bioerodable polymer and a water-soluble and/or water-dispersible organic solvent.

### (Drug with chondrogenic effect)

There are no particular restrictions on the drug with chondrogenic effect to be used in the chondrogenic composition of the invention, but it is preferably a compound represented by general formula (I) above (an indolin-2-one derivative) or a salt thereof.

Compounds represented by general formula (I) are disclosed in International Patent Publication WO94/19322, which describes such compounds as CCK-B/gastrin receptor antagonists. However, the present inventors have found that the compounds represented by general formula (I) have chondrogenic effect, and additionally that by adding such a compound to a biodegradable and/or bioerodable polymer and a water-soluble and/or water-dispersible organic solvent, it is possible to obtain a chondrogenic composition which does not require a high level of skill or sophisticated equipment for treatment, and which provides excellent biocompatibility as well as adequately high efficiency for repair and regeneration of cartilage. The compounds represented by general formula (I) may be obtained by the process described in the aforementioned International Patent Publication or by the process described in the examples provided below.

The halogen atoms and lower alkyl, lower alkenyl, lower alkynyl, lower alkoxy, acyl, aryl, lower alkylene, cycloalkyl and heterocyclic groups to be used for the invention will now be explained.

A "halogen atom" according to the invention is a fluorine atom, a chlorine atom, a bromine atom or an iodine atom.

A "lower alkyl group" according to the invention is a linear or branched alkyl group of 1 to 6 carbons, such as a methyl group, an ethyl group, a n-propyl group, an i-propyl group, a n-butyl group, a s-butyl group, a t-butyl group, a pentyl group, a hexyl group or the like.

A "lower alkenyl group" is a linear or branched alkenyl group of 2 to 6 carbons, such as a vinyl group, an allyl group, a butenyl group, a pentenyl group, a hexenyl group or the like.

A "lower alkynyl group" is a linear or branched alkynyl group of 2 to 6 carbons, such as an ethynyl group, a propynyl group, a butynyl group or the like.

A "lower alkoxy group" is. a linear or branched alkoxy group of 1 to 6 carbons, such as a methoxy group, an ethoxy group, a n-propoxy group, an i-propoxy group, a n-butoxy group, a s-butoxy group, a t-butoxy group, a pentoxy group, a hexoxy group or the like.

An "acyl group" is a carbonyl group substituted with a hydrogen atom or with an alkyl group with an optional substituent, an aryl group with an optional substituent, an alkoxy group with an optional substituent, an amino group with an optional substituent or the like, for example, an alkylcarbonyl group such as an acetyl group, a propionyl group, a pivaloyl group, a cyclohexanecarbonyl group or the like, or an arylcarbonyl group such as a benzoyl group, a naphthoyl group, a toluoyl group or the like.

An "aryl group" is a monovalent group which is an aromatic hydrocarbon minus one hydrogen atom, such as a phenyl group, a tolyl group, a xylyl group, a biphenyl group, a naphthyl group, an anthryl group, a phenanthryl group or the like.

A "lower alkylene group" is a linear or branched alkylene group of 1 to 6 carbons, such as a methylene group, an ethylene group, a propylene group, a butylene group, a pentylene group, a hexylene group or the like.

A "cycloalkyl group" is a cyclic saturated hydrocarbon group of 3 to 8 carbons, such as a cyclopropyl group, a cyclobutyl group, a cyclopentyl group, a cyclohexyl group or a cycloheptyl group. And, substituted cycloalkyl groups include menthyl group, adamantyl group and the like.

A "heterocyclic group" is an aromatic heterocyclic group with at least one hetero atom, such as a pyridyl group, a furyl group, a thienyl group, an imidazolyl group, a pyrazinyl group, a pyrimidyl group or the like.

The aforementioned lower alkyl group, lower alkenyl group, alkynyl group, lower alkoxy group, acyl group, aryl group, cycloalkyl group and heterocyclic group may, if necessary, be substituted with one or more substituents. As examples of such substituents there may be a halogen atom, a lower alkyl group, a cycloalkyl group, an aryl group, a hydroxyl group, a lower alkoxy group which alkoxy group may be substituted with a halogen atom, an aryloxy group, a lower alkylthio group, a heterocyclic group, a formyl group which formyl group may be protected as an acetal, a lower alkylcarbonyl group, an arylcarbonyl group, a carboxyl group, a lower alkoxycarbonyl group, an amino group which amino group may have a lower alkyl group, etc., an imino group, a thioacetal group, a nitro group, a nitrile group, a trifluoromethyl group and the like.

The compounds (indolin-2-one derivatives) represented by general formula (I) above will now be explained in further detail.

R¹ in formula (I) represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent, and among these, R¹ is preferably a halogen atom, a lower alkyl group, a lower alkoxy group or a nitro group.

The subscript "n" represents an integer of 0 to 4. It is preferably 0 or 1, and most preferably 0.

R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent.

R² is preferably a hydrogen atom, a lower alkyl group with an optional substituent, a lower alkenyl group with an optional substituent or an aryl group with an optional substituent, and from the viewpoint of activity as a chondrogenesis promoter, it is even more preferably a lower alkyl group with an optional substituent which is optionally substituted with a halogen atom.

Among these, R² is yet more preferably a lower alkyl group substituted at the same carbon with two lower alkoxy groups which are optionally substituted with 1-5 halogen atoms or the group -O-Z-O- wherein Z represents a lower alkylene group optionally substituted with 1-10 halogen atoms, and still more preferably, a group represented by general formula (II) : 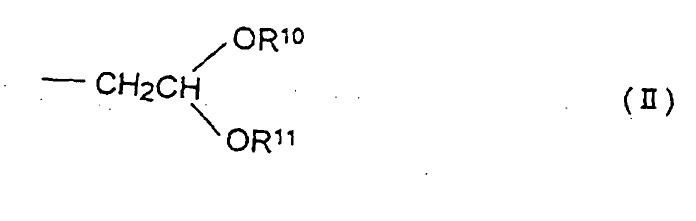 wherein R¹⁰ and R¹¹ may be the same or different, and each represents a lower alkyl group optionally substituted with 1-5 halogen atoms, preferably either or both being lower alkyl groups with 1-5 halogen atoms, or general formula (III) : 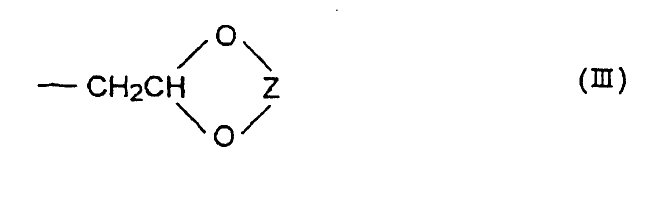 wherein Z represents a lower alkylene group optionally substituted with 1-10 halogen atoms.

R² is more preferably a 2,2-diethoxyethyl group, a 2,2-dimethoxyethyl group, a 2,2-diisopropoxyethyl group, a 2,2-bis(2-fluoroethoxy)ethyl group or a 2,2-bis(2-chloroethoxy)ethyl group, among which a 2,2-diethoxyethyl group and a 2,2-bis(2-fluoroethoxy)ethyl group are most preferred, and a 2,2-diethoxyethyl group is particularly preferred.

R³ represents a lower alkyl group with an optional substituent, a cycloalkyl group with an optional substituent, an aryl group with an optional substituent or a heterocyclic group with an optional substituent. R³ is preferably a lower alkyl group with an optional substituent, a cycloalkyl group with an optional substituent or an aryl group with an optional substituent, among which an aryl group with an optional substituent is particularly preferred. Preferred as the substituent is a lower alkyl group (preferably a methyl group and an ethyl group, and especially a methyl group) and an amino group optionally having a lower alkyl group, and a 4-methylphenyl group is especially preferred for R³.

R⁴ represents a hydrogen atom, a lower alkyl group with an optional substituent, an aryl group with an optional substituent, a heterocyclic group with an optional substituent, -OR⁵, -SR⁵ or -NR⁶R⁷. Here, R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group.

R⁴ is preferably a lower alkyl group with an optional substituent, an aryl group with an optional substituent, a heterocyclic group with an optional substituent or a group represented by -OR⁵ or -NR⁶R⁷ wherein R⁵, R⁶ and R⁷ are as previously defined, and it is more preferably the group -NR⁶R⁷ wherein R⁶ and R⁷ may be the same or different and each represents a hydrogen atom or an aryl group with an optional substituent.

Among these, R⁴ is preferably a group represented by -NR⁶R⁷ wherein R⁶ and R⁷ may be the same or different and each represents a hydrogen atom or an aryl group which aryl group has a lower alkyl group or amino group which amino group optionally has a lower alkyl group, and it is most preferably a group represented by -NHR⁷ wherein R⁷ is a 4-methylphenyl group, a 4-ethylphenyl group or a 4-(N,N-dimethylamino)phenyl group.

X and Y may be the same or different and represent -CH₂-, -NH- or -O-, among which X is preferably -CH₂-, -NH- or -O- and Y is preferably -CH₂- or -NH-. From the viewpoint of activity as a chondrogenesis promoter, X and Y may be the same or different and are preferably -CH₂- or -NH-, and most preferably, X is -NH- and Y is -CH₂-.

The indolin-2-one derivatives to be used for the invention may be used in the form of pharmaceutically acceptable salts. As examples of such salts there may be mentioned inorganic salts such as hydrochloric acid salts, hydrobromic acid salts, hydroiodic acid salts, sulfuric acid salts and phosphoric acid salts; organic acid salts such as succinic acid salts, malonic acid salts, acetic acid salts, maleic acid salts, fumaric acid salts, citric acid salts, benzoic acid salts and salicylic acid salts; and metal salts such as sodium salts, potassium salts and magnesium salts.

The indolin-2-one derivatives to be used for the invention may be in optically active forms. When in optically active forms, the absolute configuration at the 3 position is preferably the R configuration.

As examples of specific indolin-2-one derivatives to be used for the invention there may be mentioned the compounds mentioned in the examples of WO94/19322, as well as the compounds mentioned in the examples of Japanese Patent Application Laid-Open No. 7-48349, namely, Compound Nos. 1-201 listed in the following tables (Tables 1-9).

In Tables 1-9, R¹-R⁴, X, Y and n have the same definitions as for general formula (I) above.

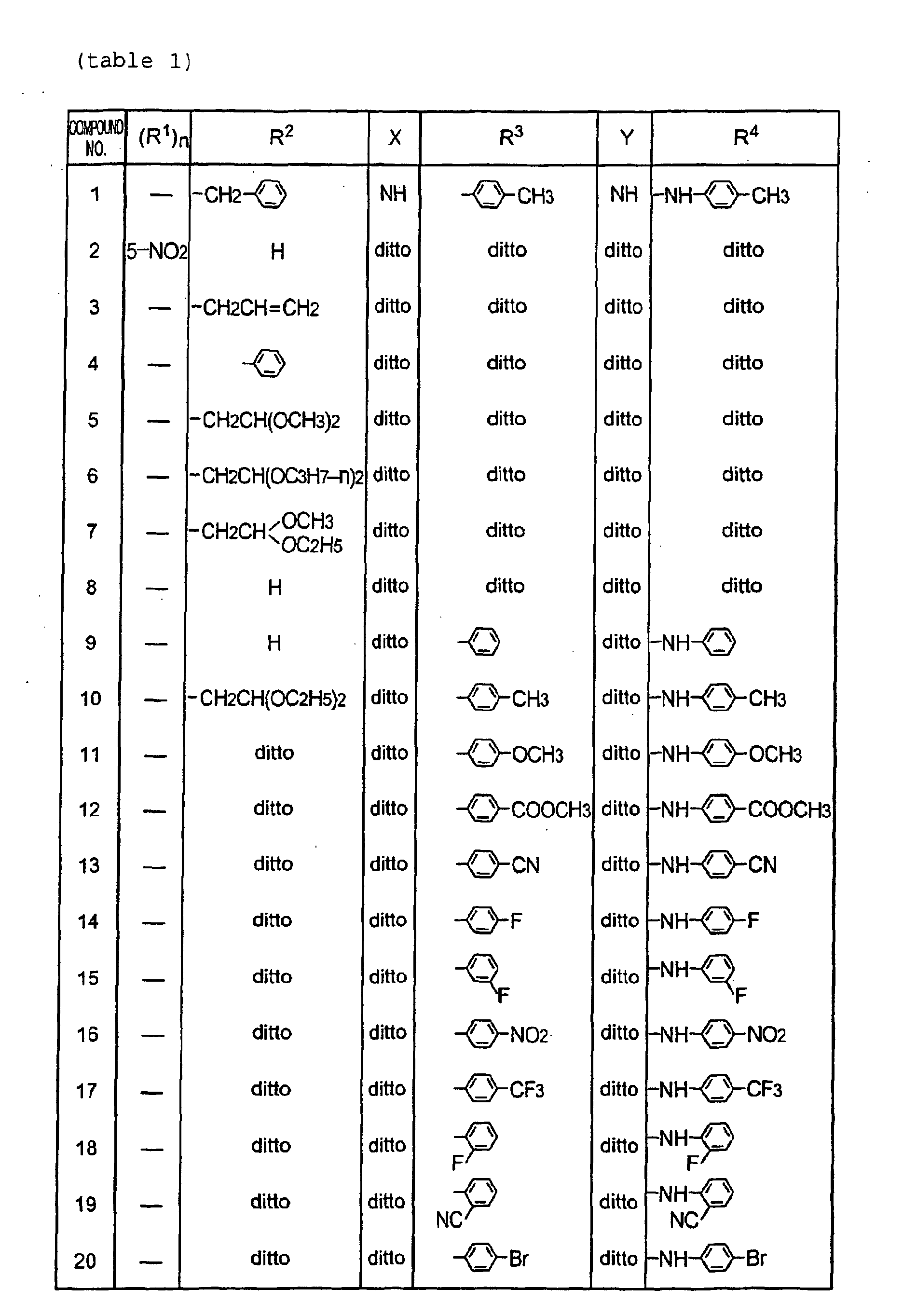

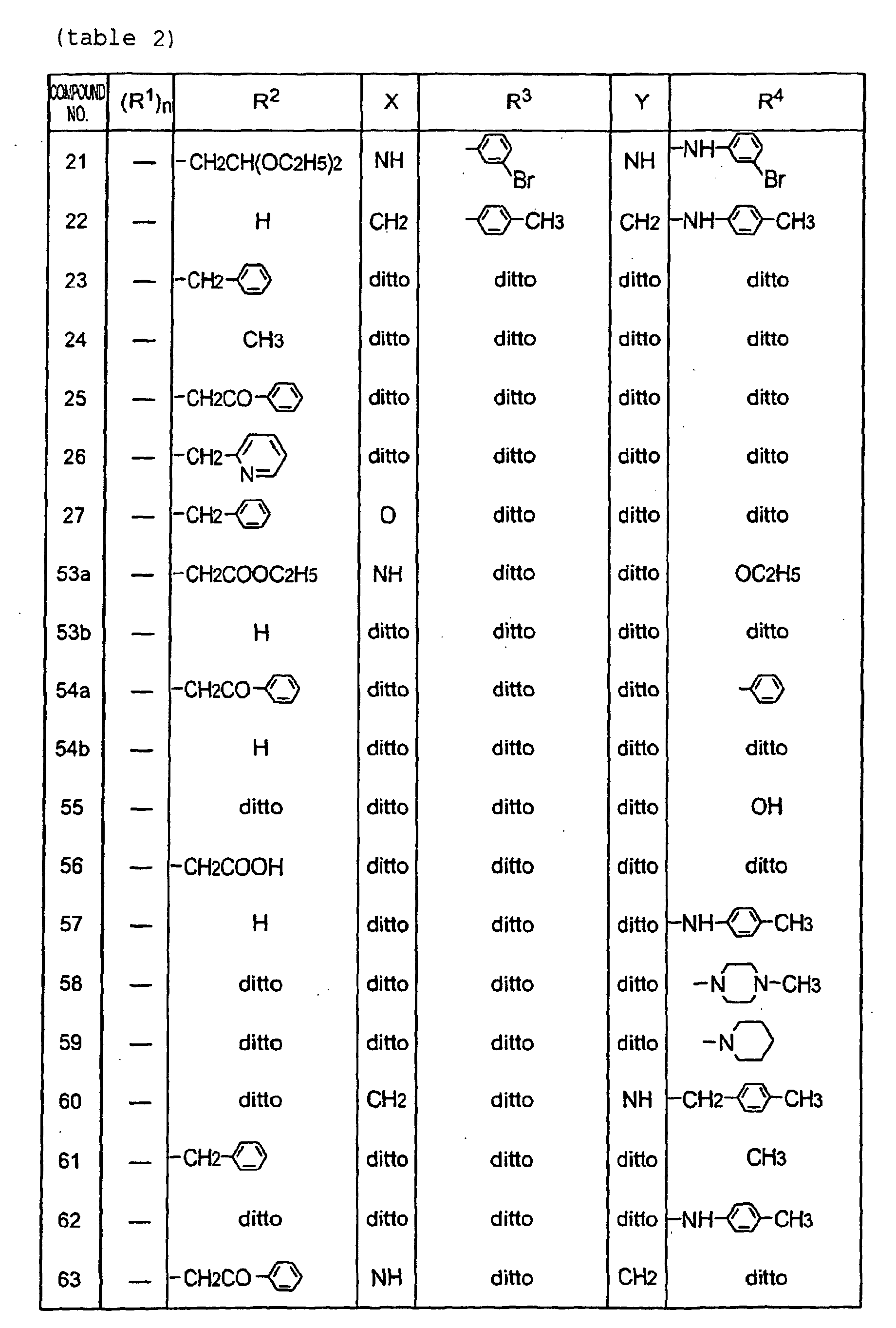

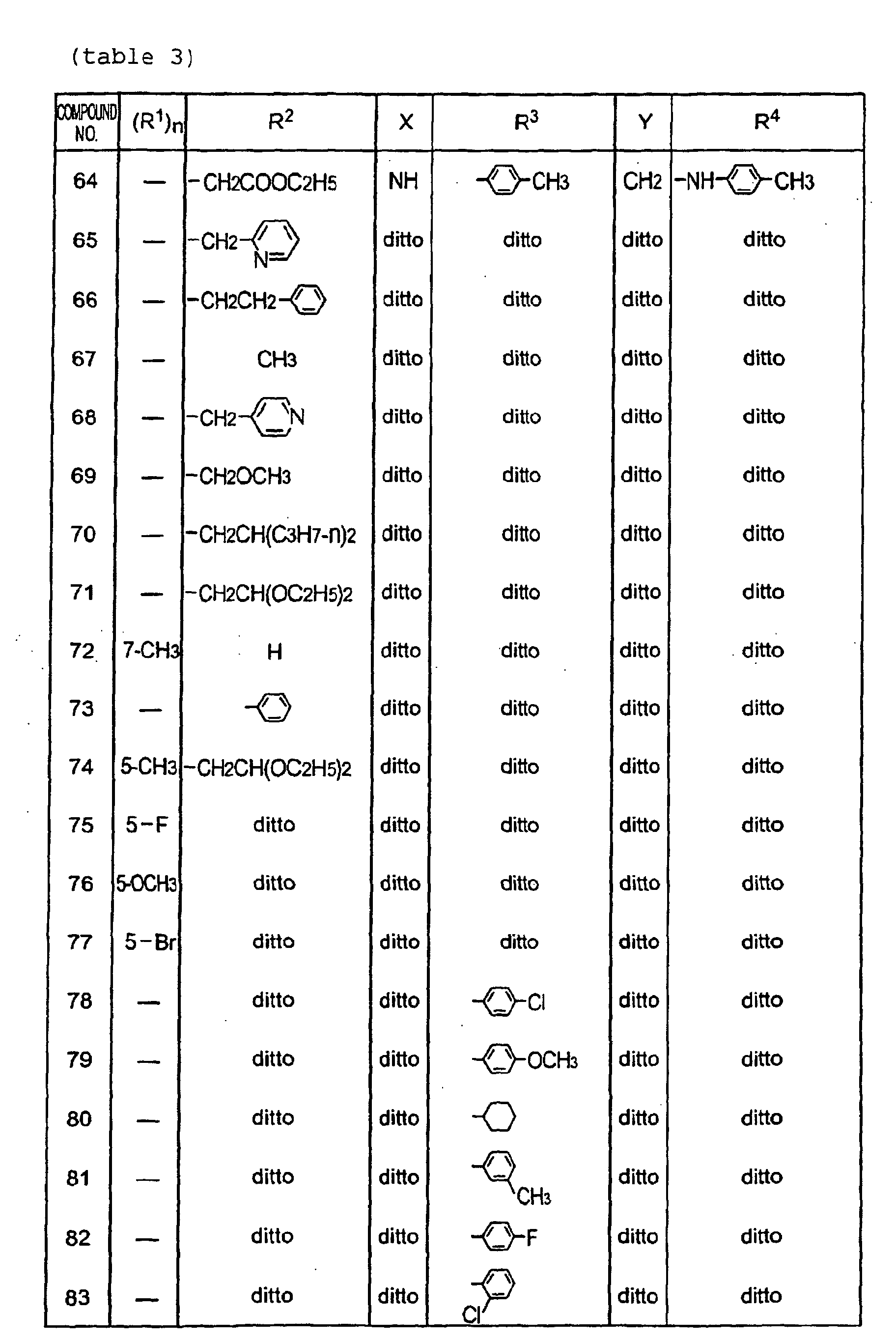

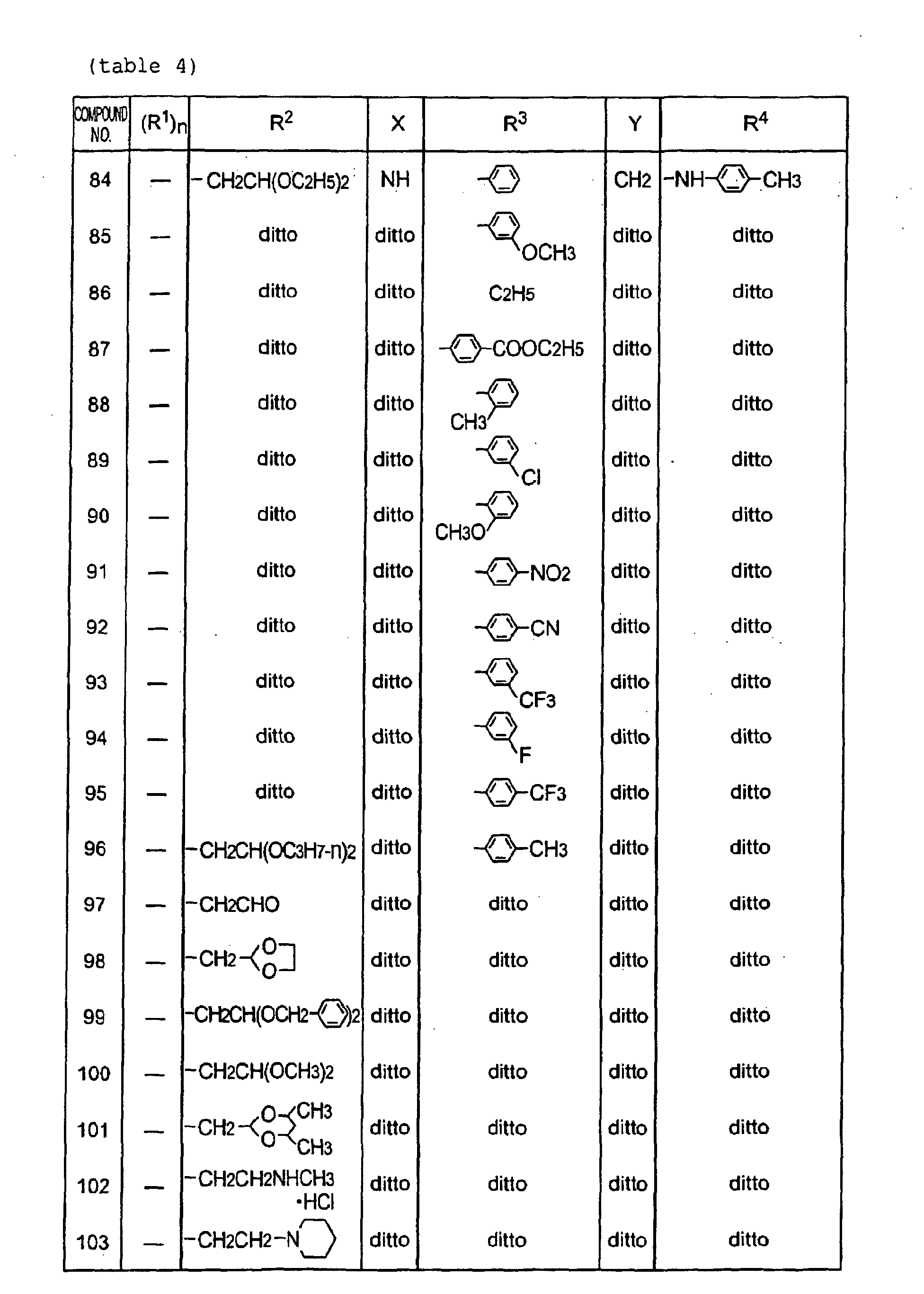

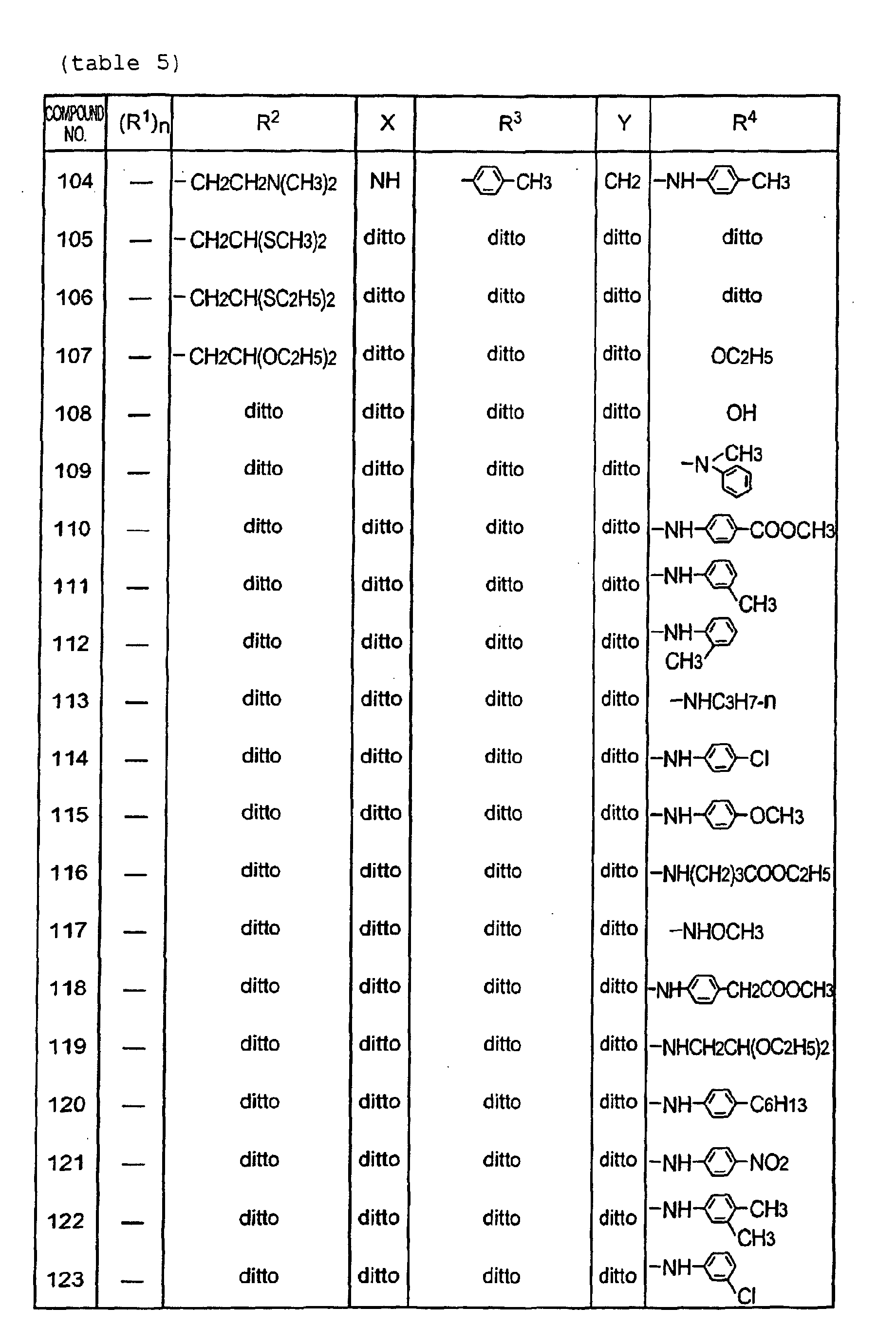

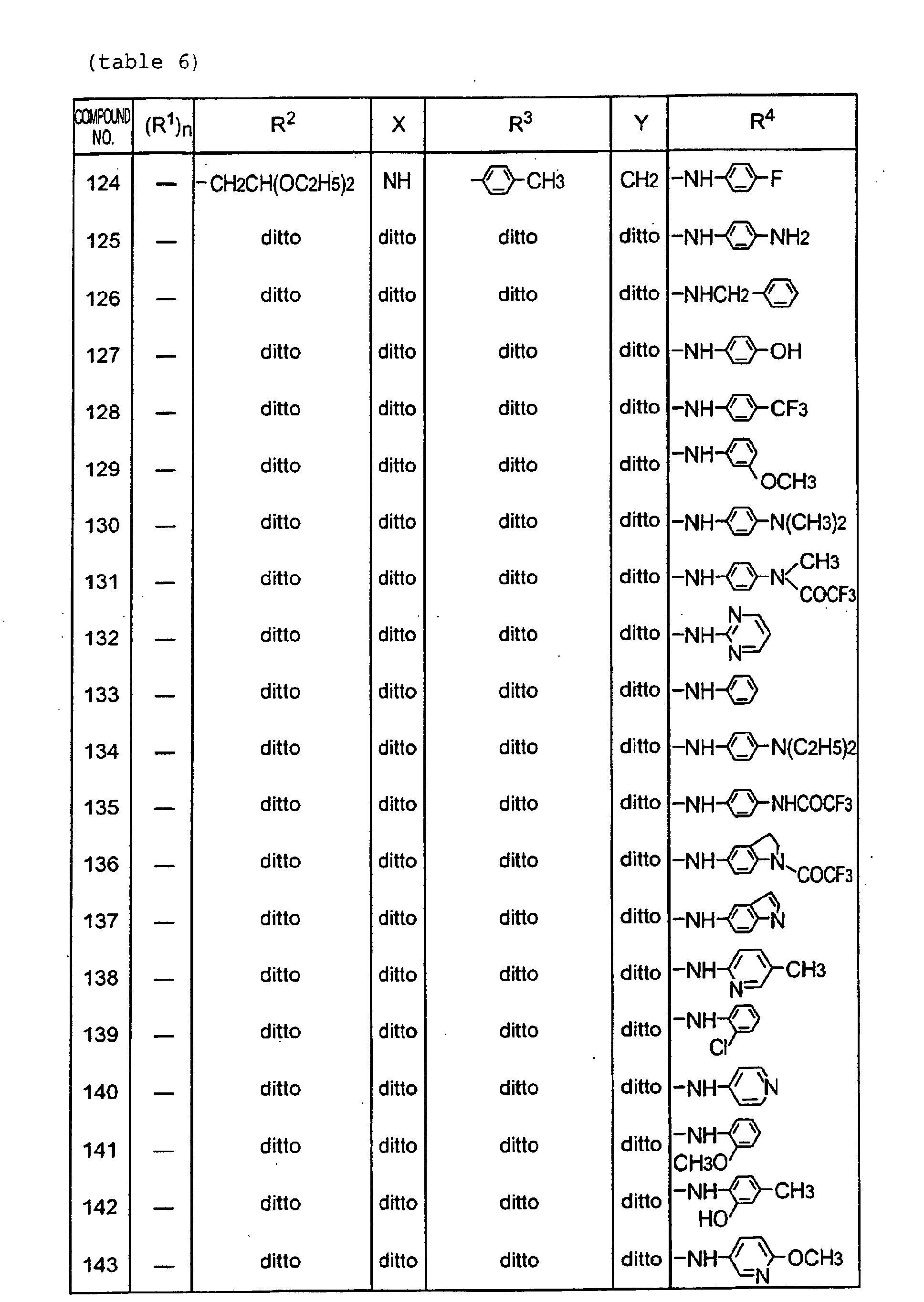

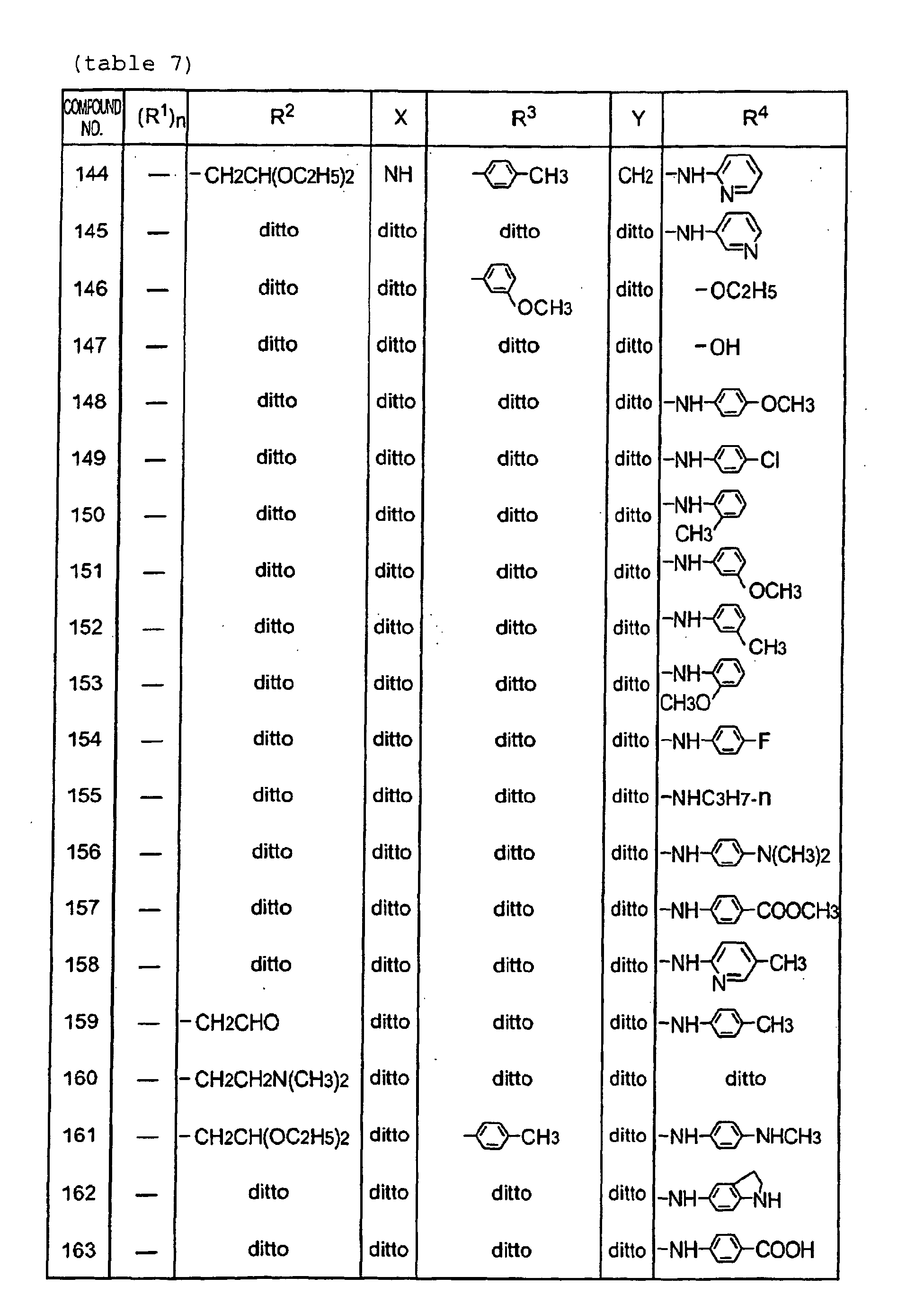

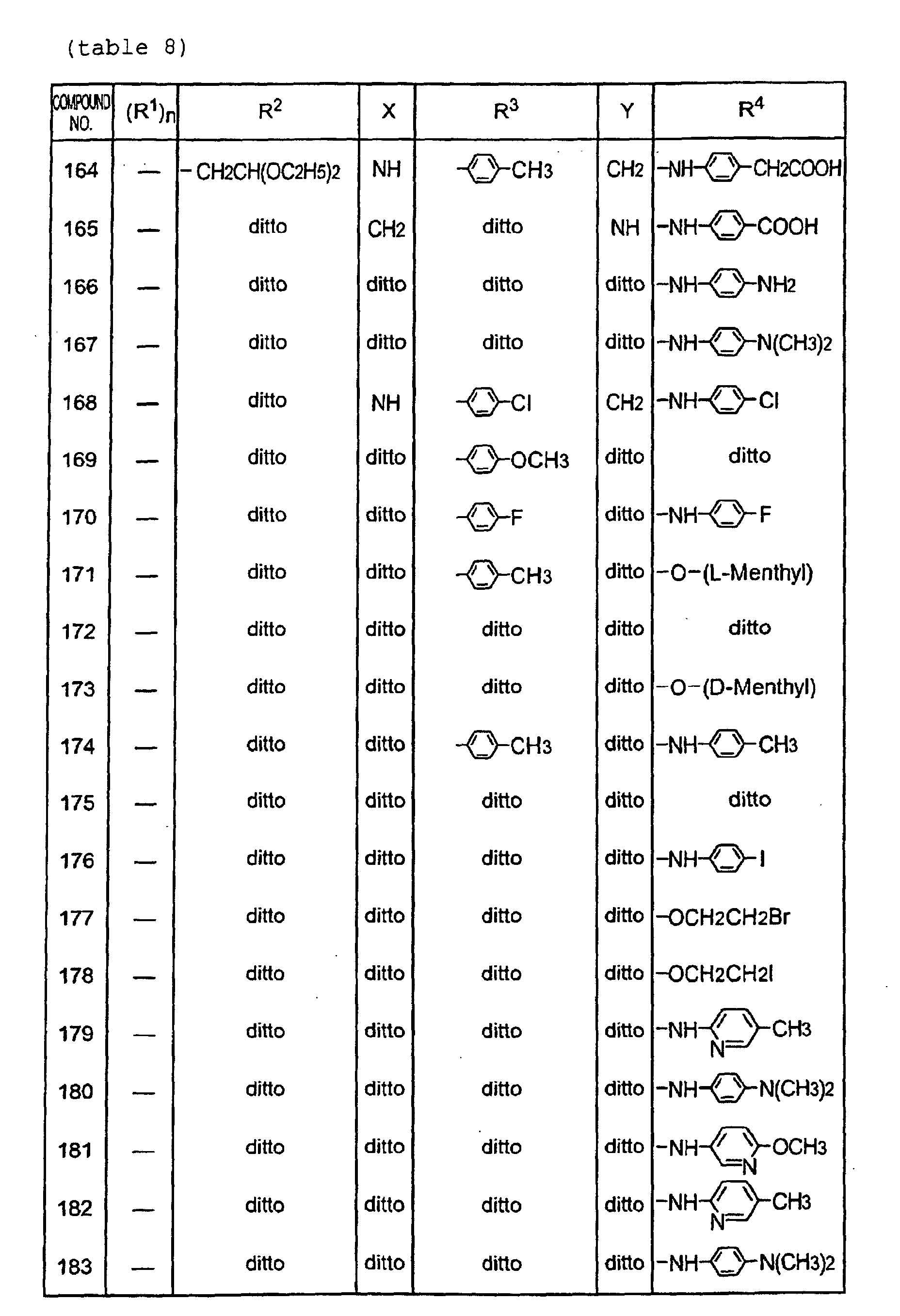

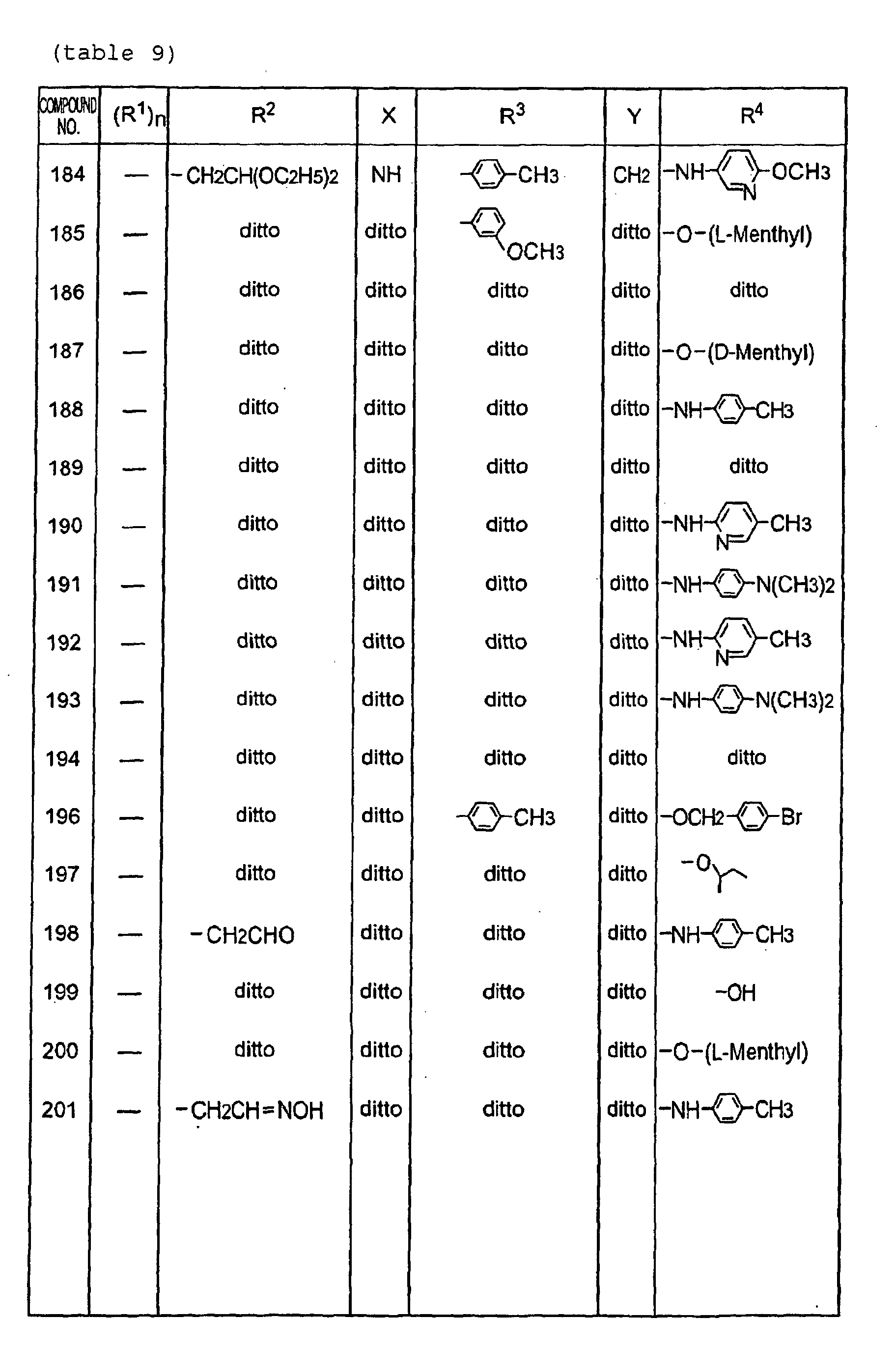

As more preferred specific indolin-2-one derivatives to be used for the invention there may be mentioned the following.

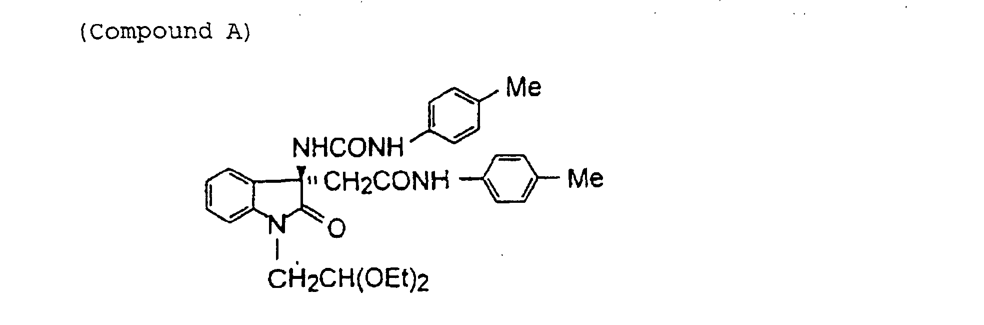

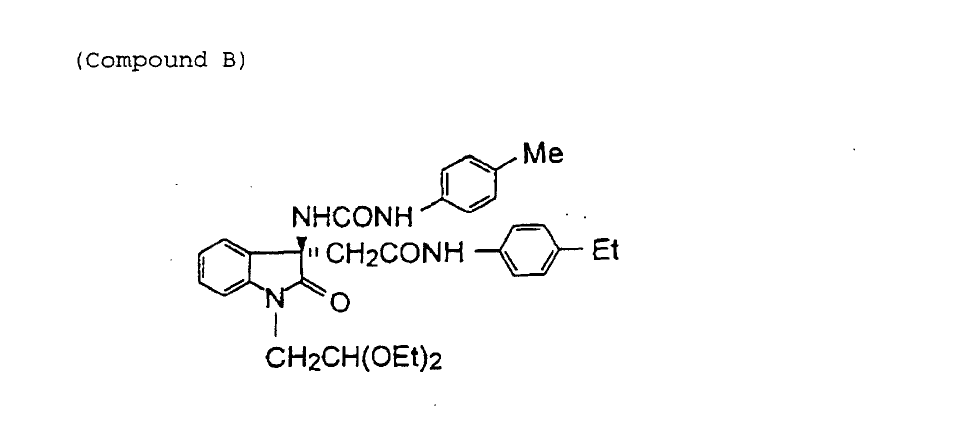

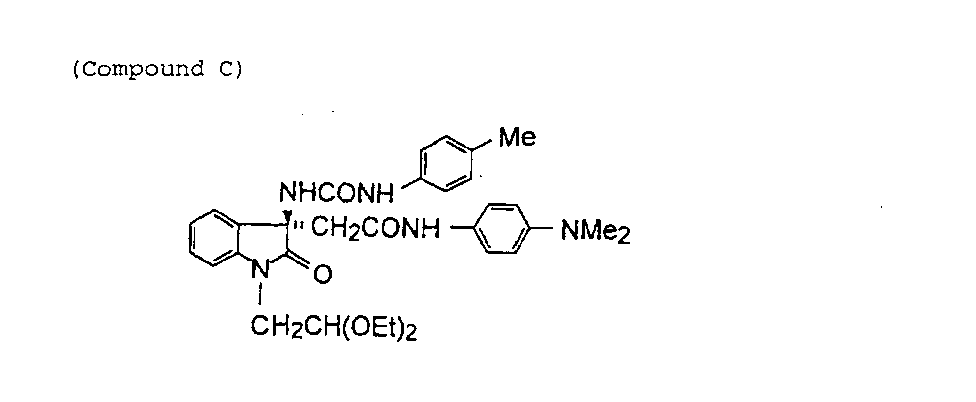

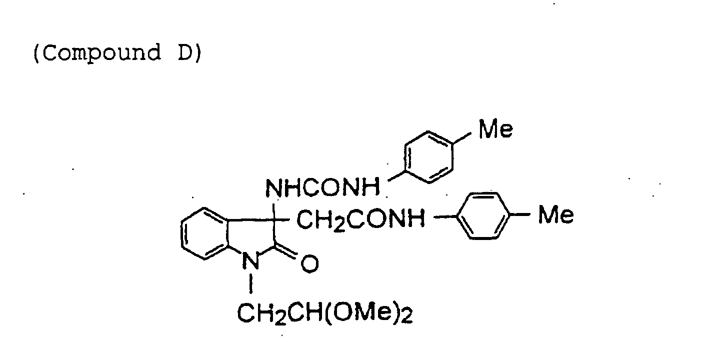

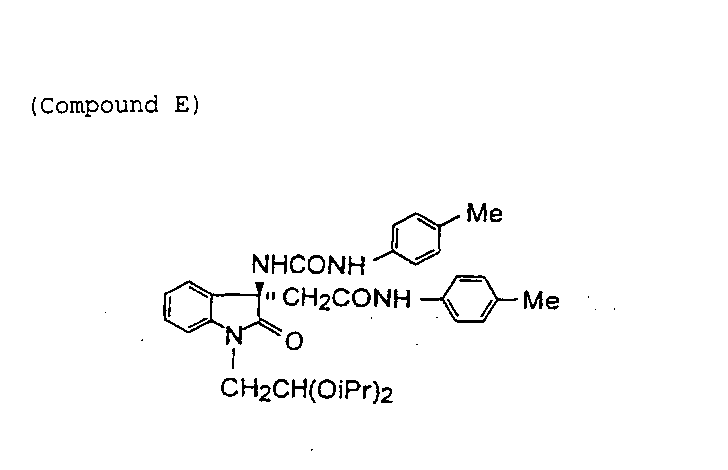

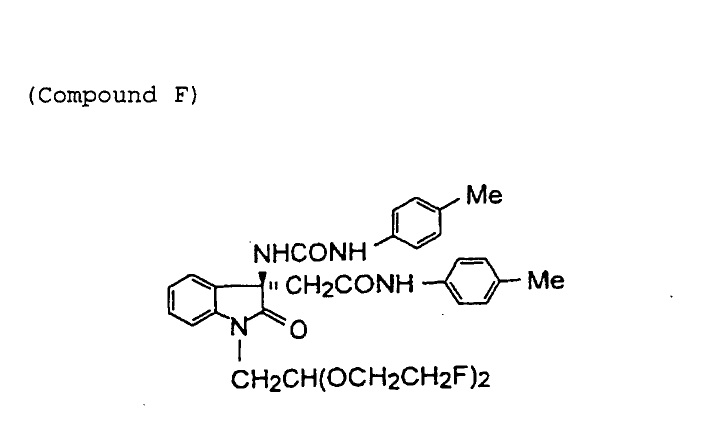

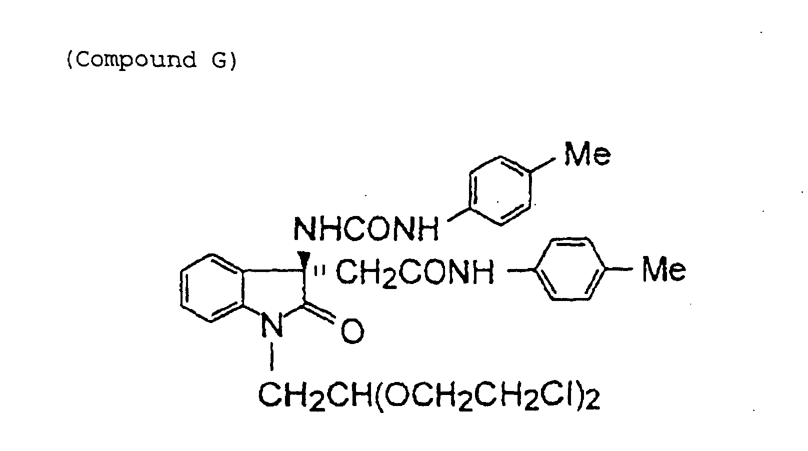

As examples of biometabolites of compound A there may be mentioned the following compounds H and I.

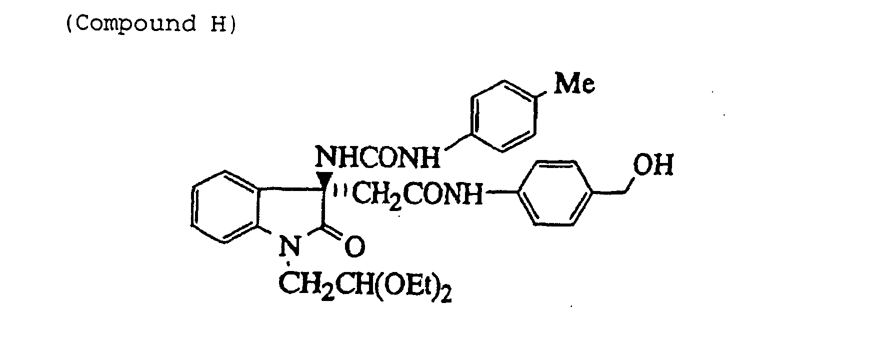

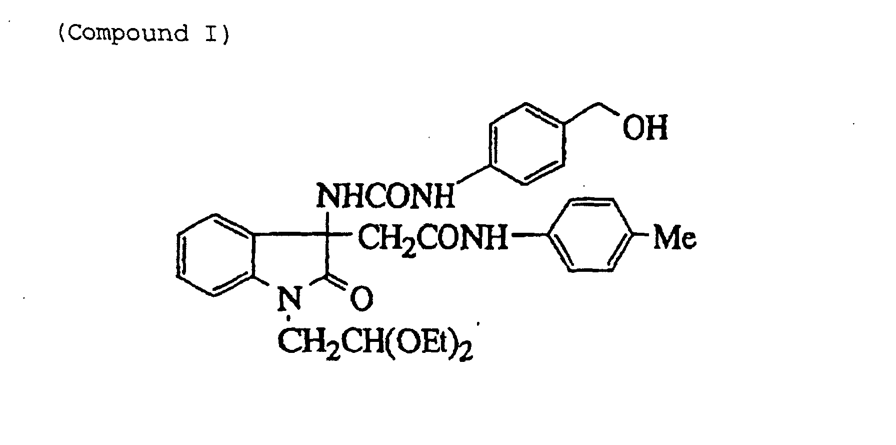

The compounds listed in Tables 1 to 9 may be synthesized by the process described in Japanese Patent Application Laid-Open No.7-48349. Of the compounds A to G mentioned above, compounds A to D may be synthesized by the process described in Japanese Patent Application Laid-Open No. 7-48349.

Compounds E, F and G may be synthesized, for example, by Reaction Path shown below (corresponding to "Reaction Path 6" in Japanese Patent Application Laid-Open No. 7-48349), using as the starting material the aldehyde intermediate mentioned in the examples of the present application, synthesized according to the process described in Japanese Patent Application Laid-Open No. 7-48349.

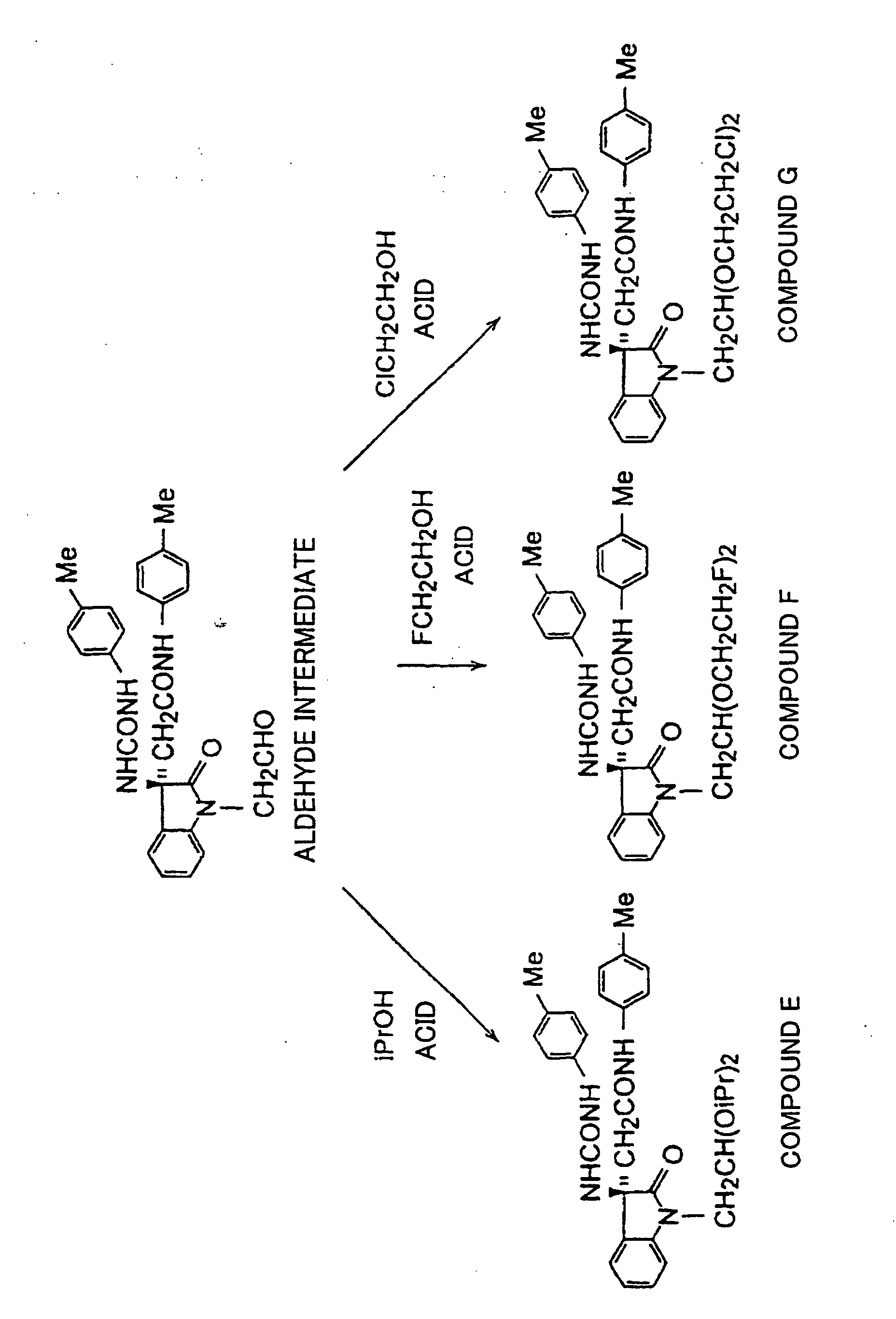

Compound H mentioned above may be synthesized according to "Reaction Path 7" in Japanese Patent Application Laid-Open No. 7-48349. Compound I may be obtained by the following process, either in a racemic form or optically active form.

Specifically, a racemic form of compound I may be synthesized, for example, according to the following Reaction Path (corresponding to "Reaction Path 5" in Japanese Patent Application Laid-Open No. 7-48349), using an isocyanate having a hydroxyl group protected with a suitable protecting group (for example, a substituted silyl group such as a triethylsilyl group, a t-butyldimethylsilyl group or the like).

An optically active form of compound I may be synthesized, for example, according to the following Reaction Path (corresponding to "Reaction Path 7" in Japanese Patent Application Laid-Open No. 7-48349), using an isocyanate having a hydroxyl group protected with a suitable protecting group (for example, a substituted silyl group such as a triethylsilyl group, a t-butyldimethylsilyl group or the like), or by optically separating a stereoisomeric mixture of compound I by a method well known to those skilled in the art (for example, a method using an optically active column).

Identification, structure determination and purity determination of the obtained compound may be accomplished by ordinary methods (spectroscopic methods such as NMR, IR, etc. and high performance liquid chromatography or the like).

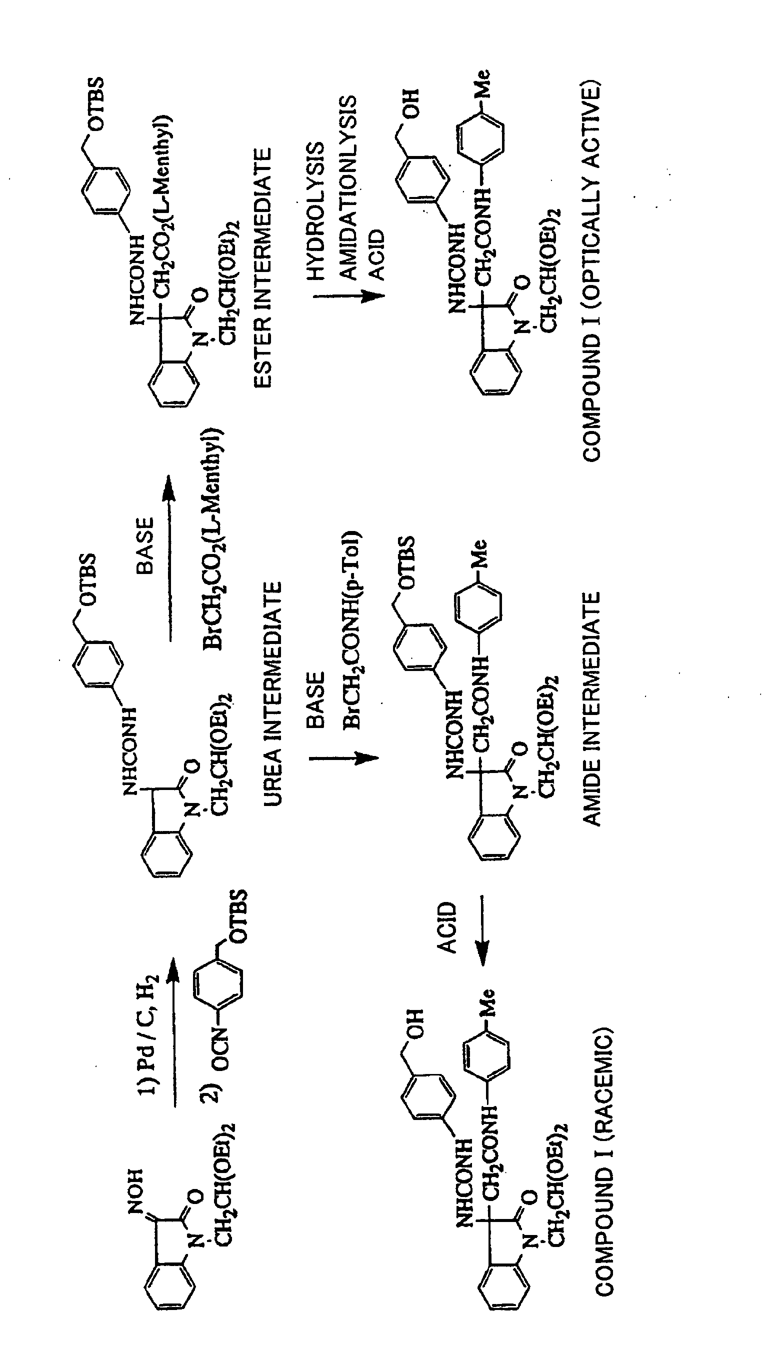

### (Biodegradable and/or bioerodable polymer)

The biodegradable and/or bioerodable polymer to be used for the invention will now be explained.

The polymer used for the invention is not particularly restricted so long as it is a biodegradable and/or bioerodable polymer. Here, "biodegradable" means the property whereby its intramolecular and/or intermolecular bonds are broken by an organism-derived enzyme (for example, a protease or esterase) or water, and "bioerodable" means the property whereby an insoluble substance is eroded in the body by chemical reaction (by enzymes or water) or physical action (incorporation by phagocytes or the like).

The polymer is mixed with the aforementioned drug with chondrogenic effect and the water-soluble and/or water-dispersible organic solvent which is described in detail later to make a chondrogenic composition. Because the polymer is dissolved, swelled or dispersed in the organic solvent together with the drug with chondrogenic effect, the chondrogenic composition shows fluidity.

When the chondrogenic composition is introduced into the body at a site of cartilage damage, for example, the organic solvent contained therein diffuses into body moisture or fluids due to its water-soluble and/or water-dispersible property. Diffusion of the organic solvent reduces the organic solvent concentration in the chondrogenic composition while body moisture or fluids also flow into the formulation, thereby increasing the moisture content of the composition. The polymer therefore begins to gradually precipitate as it solidifies and/or gels. Most of the drug with chondrogenic effect stays in the gap of the damaged cartilage together with the precipitated polymer. Because the precipitated polymer is biodegradable and/or bioerodable, it undergoes gradual decomposition (low molecularization) and/or erosion in the body, resulting in gradual release of the drug. In effect, therefore, the chondrogenic composition of the invention shows a sustained-release of drug with chondrogenic effect. The released drug stimulates proliferation of chondrocytes, which use the precipitated polymer as a scaffold for attachment.

The polymer used for the invention having the function described above is therefore preferably a porous body-forming polymer to hold the chondrocytes which have proliferated in response to the drug with chondrogenic effect. That is, it is preferably a polymer which precipitates as a porous body due to changes in the organic solvent concentration or moisture content of the chondrogenic composition. If the precipitated polymer is porous, the size and number of the pores may be controlled to regulate release of the drug with chondrogenic effect. Furthermore, since the chondrocytes may then attach in the numerous fine microvoids, the efficiency of cartilage repair and regeneration is increased.

The polymer used for the invention preferably has a weight-average molecular weight of 500 or greater, and more preferably a weight-average molecular weight of 500-200,000. Most preferably, the weight-average molecular weight of the polymer is 1,000-100,000. If the weight-average molecular weight of the polymer is less than 500, the strength of the polymer precipitated from the chondrogenic composition will tend to be lower. On the other hand, if the weight-average molecular weight of the polymer used for the invention is greater than 200,000, the flow property of the chondrogenic composition will tend to be lower.

The chondrogenic composition of the invention has a sustained release property of the drug as explained above, and the sustained release property can be controlled by altering the type or molecular weight of the polymer. For example, a polymer with a higher biodegradable and/or bioerodable property will give a faster drug release rate, while a larger molecular weight polymer, having higher hydrophobicity, will exhibit reduced water permeability and a reduced biodegradable and/or bioerodable property, thereby slowing the drug release rate.

As polymers for the invention there are preferably used polyhydroxyalkanoic acids, lactone ring-opened polymers, hydroxyalkanoic acid/lactone copolymers, polyacetals, polyketals, polymethylvinyl ether, chitin, chitosan, polyamides, polyamino acids, polyurethane, polyester amides, polycarboxylic acids, polyanhydrides, polyalkylenes, polyphosphoric acid, polyortho esters or copolymers containing at least one of these in the molecule. These polymers may be used alone or in combinations of two or more.

As polyhydroxyalkanoic acids there may be mentioned hydroxyalkanoic acid polymers such as glycolic acid polymer (polyglycolic acid), lactic acid polymer (polylactic acid), hydroxybutyric acid polymer (polyhydroxybutyric acid) and hydroxyvaleric acid polymer (polyhydroxyvaleric acid). As hydroxyalkanoic acid copolymers there may be mentioned lactic acid/glycolic acid copolymer, hydroxybutyric acid/hydroxyvaleric acid copolymer, and the like. These polyhydroxyalkanoic acids and hydroxyalkanoic acid copolymers may be substituted, and preferably contain 12 or fewer carbon atoms.

As lactone ring-opened polymers (polymers made by ring-opening polymerization of lactone ) there may be mentioned butyrolactone ring-opened polymer (polybutyrolactone), valerolactone ring-opened polymer (polyvalerolactone), caprolactone ring-opened polymer (polycaprolactone), dioxanone ring-opened polymer (polydioxanone) and the like, and as hydroxyalkanoic acid/lactone copolymers there may be mentioned copolymers of the above-mentioned hydroxyalkanoic acids and lactones, such as glycolic acid/lactone copolymer and lactic acid/valerolactone copolymer.

There are no particular restrictions on polyamides that may be used so long as they are reaction products of polyamines and polycarboxylic acids and are biodegradable and/or bioerodable, but they are preferably reaction products of aliphatic polyamines of 12 or fewer carbons and aliphatic polycarboxylic acids of 12 or fewer carbons. The term "polyamino acids" refers to polymers of aminocarboxylic acids (amino acids) and includes oligopeptides and polypeptides (proteins).

There are no particular restrictions on polyurethanes that may be used so long as they are reaction products of polyols and polyisocyanates and are biodegradable and/or bioerodable, but they are preferably reaction products of aliphatic polyols of 12 or fewer carbons and isocyanates of 12 or fewer carbons.

There are no particular restrictions on polyester amides that may be used so long as they have both an ester bond and an amide bond in the molecule and are biodegradable and/or bioerodable, but they preferably have a polyester skeleton composed of an aliphatic polyol of 12 or fewer carbons and a polycarboxylic acid of 12 or fewer carbons, and a polyamide skeleton composed of an aliphatic polyamine of 12 or fewer carbons and an aliphatic polycarboxylic acid of 12 or fewer carbons.

As polycarboxylic acids there may be mentioned polymers of compounds containing two or more carboxyl groups such as polymaleic acid, polymalic acid and polyglutamic acid, and as polyanhydrides there may be mentioned polymers having carboxylic anhydride structures such as polysebacic anhydride and polymaleic anhydride. As polyalkylenes there may be mentioned polymers having alkylene groups and ester groups, such as polyalkylene oxalic acid esters and polyalkylene succinic acid esters.

As polyphosphoric acids there may be mentioned linear polymers produced by dehydrated condensation of orthophosphoric acid, and as a polyortho ester there may be mentioned 3,9-bis(ethylidene-2,4,8,10-tetraoxaspiro[5.5]undecane based polymer.

According to the invention, a reactive polymer having an unsaturated double bond may be used for at least a portion of the polymer. A reactive polymer having an unsaturated double bond will produce a polymerization reaction upon mixture with a polymerization catalyst, heating or exposure to light. When using a reactive polymer with two or more unsaturated double bonds, three-dimensional crosslinking will be produced by the polymerization reaction.

As reactive polymers there are preferred compounds comprising a skeleton composed of at least one polymer selected from the group consisting of polyhydroxyalkanoic acids, lactone ring-opened polymers, hydroxyalkanoic acid/lactone copolymers, polyacetals, polyketals, polymethylvinyl ether, chitin, chitosan, polyamides, polyamino acids, polyurethane, polyester amides, polycarboxylic acids, polyanhydrides, polyalkylenes, polyphosphoric acid, polyortho esters and copolymers containing at least one of these in the molecule, with at least one (meth)acryloyl group bonded to the skeleton. Here, "(meth)acryloyl group" means "acryloyl group or methacryloyl group".

The compounds mentioned above may be used as polyhydroxyalkanoic acids, lactone ring-opened polymers, hydroxyalkanoic acid/lactone copolymers, polyacetals, polyketals, polymethylvinyl ether, chitin, chitosan, polyamides, polyamino acids, polyurethane, polyester amides, polycarboxylic acids, polyanhydrides, polyalkylenes, polyphosphoric acid and polyortho esters for the skeleton of the reactive polymer.

There are no particular restrictions on the positions or number of (meth)acryloyl groups bonded to the skeleton, but (meth)acryloyl groups are preferably bonded to at least one end of the skeleton.

When a (meth)acryloyl group is bonded to the end of a polyester such as a polyhydroxyalkanoic acid, lactone ring-opened polymer or hydroxyalkanoic acid/lactone copolymer, a (meth)acryloyloxy bond ((meth)acrylic acid ester bond) is formed.

When the polymer used in the chondrogenic composition of the invention contains one of the aforementioned reactive polymers, a polymerization initiator for the reactive polymer is preferably added to the chondrogenic composition before use. From the standpoint of stability of the chondrogenic composition over time, the polymerization initiator is preferably added just prior to use. The polymerization initiator used is not particularly restricted, and for example, there may be mentioned azo compounds such as azobisisobutyronitrile (AIBN), peroxides such as benzoyl peroxide, two-component redox initiators such as benzoyl peroxide-dimethylaniline, and photoinitiators such as α,α-dimethoxy-α-phenylacetophenone (DMPA).

Since the chondrogenic composition of the invention is to be used in the body, the polymerization initiator preferably has catalytic activity at near body temperature or below body temperature.

### (Water-soluble and/or water-dispersible organic solvent)

The water-soluble and/or water-dispersible organic solvent to be used for the invention will now be explained.

The organic solvent to be used for the invention may be any one which is water-soluble and/or water-dispersible, with no particular restrictions. Preferred organic solvents include N-methyl-2-pyrrolidone, 2-pyrrolidone, ethanol, benzyl alcohol, propyleneglycol, acetone, ethyl acetate, methyl ethyl ketone, dimethylformamide, dimethylsulfoxide, tetrahydrofuran, caprolactam, N,N-dimethylacetamide, triacetin, glycerol, polyoxyethylene-hardened castor oil, vitelline phospholipid, soy lecithin, polyoxyethylene sorbitan fatty ester and polyoxyethylene alkylether. These solvents may be used alone or in combinations of two or more.

As mentioned above, the organic solvent has the function of dissolving, swelling or dispersing the polymer and drug with chondrogenic effect, and when the chondrogenic composition is introduced into the body at a site of cartilage damage, it has the function of diffusing into body moisture or fluids to precipitate the polymer. According to the invention, N-methyl-2-pyrrolidone is most preferably used as the organic solvent because of its superiority in exhibiting such functions.

### (Water-soluble compound)

The chondrogenic composition of the invention may also contain a water-soluble compound in addition to the aforementioned drug with chondrogenic effect, polymer and organic solvent. A "water-soluble compound" means a compound which is soluble in body moisture or fluids.

As mentioned above, when the chondrogenic composition is introduced into the body at a site of cartilage damage, the organic solvent in the chondrogenic composition diffuses into body moisture or fluids, thereby reducing the organic solvent concentration in the chondrogenic composition while body moisture or fluids also flow into the composition, thereby increasing the moisture content of the composition.

When the chondrogenic composition contains a water-soluble compound, the water-soluble compound affects the flow volume or flow rate of the moisture or fluid thus possibly altering the form of the precipitating polymer, and altering the sustained release of the drug with chondrogenic effect and the efficiency of repair and regeneration of cartilage. Particularly when using a polymer which is porous when precipitated, it becomes possible to alter the sizes of the pores while also adjusting the drug release rate. Specifically, the pore size tends to increase with a greater water-soluble compound content, and a chondrogenic composition containing the water-soluble compound tends to have a higher drug release rate than one containing no such compound.

The water-soluble compound preferably has a mean particle size of 1-3000 µm. The mean particle size is more preferably 50-600 µm and even more preferably 100-400 µm. If the mean particle size is less than 1 µm, then the pore size-controlling effect tends to be lower when using a polymer that can form a porous body by precipitation, whereas if the mean particle size is greater than 3000 µm, the pore size becomes too large when using a polymer that can form a porous body by precipitation, and therefore the chondrogenic effect will sometimes be reduced.

The water-soluble compound used for the invention is preferably a compound that exhibits a pH of 6 or greater in aqueous solution either alone or in a combination of two or more. Because the polymer used according to the invention is biodegradable and/or bioerodable, the polymer gradually decomposes (low molecularizes) in the body. Since most of the decomposition product consists of acidic substances, it is possible to control pH reduction of the chondrogenic composition in the body by using water-soluble compound that can produce a pH of 6 or greater in aqueous solution when used alone or in combinations of two or more. Consequently, the stability of the drug with chondrogenic effect in the chondrogenic composition can be improved. Its irritation of the body can also be attenuated.

When the water-soluble compound for the invention is used alone or as a combination of two or more, it is preferably a compound which produces a pH of 7 or greater and more preferably a compound that produces a pH of 7-9, in aqueous solution. Also, when the water-soluble compound is used alone or as a combination of two or more, it is most preferably a compound with a buffering effect which keeps the pH of the chondrogenic composition in the range of 7-8 when introduced into the body.

As water-soluble compounds there may be mentioned water-soluble inorganic compounds such as monosodium phosphate, monosodium phosphate hydrate, disodium phosphate, disodium phosphate hydrate, trisodium phosphate, trisodium phosphate hydrate, magnesium sulfate, magnesium sulfate hydrate, magnesium sulfate double salts, magnesium hydroxide and sodium chloride; saccharides such as glucose, mannitol, xylose, sucrose, fructose, hyaluronic acid and dextran; amino acids; polypeptides; and the like. These water-soluble compounds may be used alone or in combinations of two or more. As preferred examples of combinations of two or more there may be mentioned a mixture of monosodium phosphate and disodium phosphate in a mixing ratio to give a pH of 6 or greater in aqueous solution.

The content of the aforementioned water-soluble compound in the chondrogenic composition is preferably no greater than 90 wt% and more preferably 10-80 wt%. The content is even more preferably 50-75 wt%. If the content of the water-soluble compound exceeds 90 wt%, the relative content of the biodegradable and/or bioerodable compound is reduced, tending to lower the efficiency of cartilage repair and regeneration.

### (Chondrogenic composition)

The chondrogenic composition of the invention will now be explained. The chondrogenic composition of the invention will be adequate if it comprises a drug with chondrogenic effect, a biodegradable and/or bioerodable polymer and a water-soluble and/or water-dispersible organic solvent. The chondrogenic composition of the invention may also contain the aforementioned water-soluble compound, and so long as its function as a formulation for chondrogenesis is not impaired, it may still further contain collagen, gelatin or the like. When ethanol is not included as the organic solvent, it may still further include ethanol. The chondrogenic composition of the invention will exhibit excellent biocompatibility as a consequence of comprising the aforementioned components.

There are no particular restrictions on the process for producing the chondrogenic composition of the invention, and for example, the drug with chondrogenic effect and the biodegradable and/or bioerodable polymer may be mixed with the water-soluble and/or water-dispersible organic solvent under conditions in which they are not denatured. Here, the drug with chondrogenic effect and the polymer may be dissolved, swelled or dispersed by mixture with the organic solvent, and the chondrogenic composition of the invention need only be fluid at least at the time of use.

According to the invention, the drug with chondrogenic effect content in the chondrogenic composition is preferably 0.0001-50 wt%, more preferably 0.0001-25 wt% and even more preferably 0.0001-10 wt%.

The polymer content according to the invention is preferably 5-80 wt%, more preferably 10-70 wt% and even more preferably 25-50 wt%.

The organic solvent content according to the invention is preferably 5-90 wt%, more preferably 5-80 wt% and even more preferably 5-70 wt%.

The proportion of the drug with chondrogenic effect with respect to the polymer (drug weight/polymer weight) is preferably 0.000001-0.9, more preferably 0.00001-0.5 and even more preferably 0.0001-0.2. The proportion of the organic solvent with respect to the polymer (organic solvent volume/polymer weight) is preferably 0.1-10, more preferably 0.2-4 and even more preferably 0.3-2. When the aforementioned water-soluble compound (or water-soluble compound mixture) is used according to the invention, the proportion of the water-soluble compound with respect to the polymer (total water-soluble compound weight/polymer weight) is preferably 0.01-40, more preferably 0.1-30 and even more preferably 1-30.

As explained above, the chondrogenic composition of the invention has a sustained drug release property. The drug release period and release pattern may be controlled by altering the solubility or dispersability of the organic solvent of the formulation into moisture or body fluids, the dispersability of moisture or body fluids into the formulation containing the solidified or gelled polymer, and the biodegradable and/or bioerodable property of the polymer. The drug release period and release pattern are also controlled by altering the compositional ratio of the components in the formulation and the hydrophobicity or decomposition property which are governed by the molecular weight or composition of the polymer.

According to the invention, when using a polymer which forms a porous body it is possible to control release period of the drug with chondrogenic effect and release pattern by altering the size (mean diameter) or number of the pores. The mean diameter is preferably 3-3000 µm, more preferably 50-600 µm and even more preferably 100-400 µm.

The chondrogenic composition of the invention may be used as a formulation for direct embedding into damaged cartilage, or as a coating formulation for direct coating of damaged cartilage. Alternatively, it may be used as an injection formulation to be administered to the body via injection, or as an endoscopic formulation to be administered to the body via an endoscope. By employing it in such a manner, it serves as a therapeutic agent for repair and regeneration of damaged cartilage in the body of a patient with congenital or traumatic cartilage damage. In addition, by placing and culturing chondrocytes in the solidified chondrogenic composition it is possible to form cartilage *ex vivo,* in order to allow application for treatment by formation of cartilage of the necessary shape from autocartilage cells and transplanting into the body. Thus, the chondrogenic composition of the invention can be utilized for application in repair, regeneration and formation of cartilage both *in vivo* and *ex vivo* in the field of regenerative medical engineering (tissue engineering), a currently thriving field of research.

The present invention will now be explained in further detail by way of preferred examples, with the understanding that these examples are not limitative on the invention.

### (Examples)

### (Synthesis of drug with chondrogenic effects)

### Synthesis Example 1

Compounds A, C and D were synthesized by the following methods.

### Compound A

Compound A was synthesized by the method described in "Example 174(2)" of Japanese Patent Application Laid-Open No. 7-48349. The racemic form of this compound was synthesized by the method described in "Example 71" of the same publication.

### Compound C

Compound C was synthesized by the method described in "Example 180" of Japanese Patent Application Laid-Open No. 7-48349.

### Compound D

Compound D was synthesized by the method described in "Example 100" of Japanese Patent Application Laid-Open No. 7-48349.

### Synthesis Example 2

Compounds B, E, F and G were synthesized by the following methods.

### Compound B (3R)-1-(2,2-diethoxyethyl)-3-(4-ethylphenyl)aminocarbonylmethyl-3-(N'-(4-methylphenyl)ureido)indolin-2-one

(+)-1-(2,2-diethoxyethyl)-3-hydroxycarbonylmethyl-3-(N'-(4-methylphenyl)ureido)indolin-2-one (compound described in "Example 174(1)" of Japanese Patent Application Laid-Open No. 7-48349, 182 mg) was dissolved in dichloromethane (10 mL), and then 1-ethyl-3-(3-dimethylaminopropyl)-carbodiimide hydrochloride (96 mg) and paraethylaniline (61 mg) were added. The obtained mixture was stirred at 15-30°C for 15 minutes.

The resulting reaction mixture was consecutively washed with 1N dilute hydrochloric acid and saturated sodium bicarbonate water, and after drying over anhydrous sodium sulfate (15-30°C), the solvent was removed off under reduced pressure for concentration. The residue was purified by silica gel column chromatography (hexane/ethyl acetate = 3/1) to obtain 145 mg of the title compound as a white powder (65% yield).
NMR(CDCl₃, 270 MHz)
δ 8.48(brs, 1H), 7.37-6.82(m, 14H), 4.77(t, J=5.0 Hz, 1H), 3.94(dd, J=5.8,13.7 Hz, 1H), 3.80-3.42(m, 5H), 3.00(d, J=14.7 Hz, 1H), 2.66(d, J=14.7 Hz, 1H), 2.53(q, J=7.6 Hz, 2H), 2.17(s, 3H), 1.20-1.05(m, 9H).

### Aldehyde intermediate

### (3R)-1-(formylmethyl)-3-((4-methylphenyl)aminocarbonylmethyl)-3-(N'-(4-methylphenyl)ureido)indolin-2-one

To a solution of (+)-1-(2,2-diethoxyethyl)-3-((4-methylphenyl)aminocarbonylmethyl)-3-(N'-(4-methylphenyl)ureido)indolin-2-one (compound described in "Example 174(2)" of Japanese Patent Application Laid-Open No. 7-48349, 610 mg) in acetone (30 mL) there were added water (10 mL) and concentrated hydrochloric acid (3 mL), and the mixture was heated to reflux for 2 hours. After cooling the reaction solution, the solvent was removed off under reduced pressure for concentration. Dichloromethane was added to the residue and washing was performed twice with saline solution. After drying the organic layer over anhydrous magnesium sulfate (15-30°C), it was concentrated under reduced pressure to obtain 529 mg of the title compound as a crude product.
NMR(CDCl₃, 270 MHz)
δ 9.71 (s, 1H), 7.75(s, 1H), 7.40(s, 1H), 7.38-6.90(m, 11H), 6.65(d, J=7.9 Hz, 1H), 4.76(d, J=18.7 Hz, 1H), 4.40(d, J=18.7 Hz, 1H), 2.98(d, J=14.5 Hz, 1H), 2.55(d, J=14.5 Hz), 2.30(s, 3H), 2.23 (s, 3H).

### Compound E

### (3R)-1-(2,2-diisopropoxyethyl)-3-((4-methylphenyl)aminocarbonylmethyl)-3-(N'-(4-methylphenyl)ureido)indolin-2-one

The aldehyde intermediate (60 mg) obtained above was dissolved in isopropanol (10 mL), and then paratoluenesulfonic acid (10 mg) was added and the mixture was heat to reflux for 4 hours. After allowing the reaction solution to cool, the solvent was removed off under reduced pressure for concentration. The residue was diluted with dichloromethane and washed with saturated sodium bicarbonate water. The organic layer was dried (15-30°C) over anhydrous magnesium sulfate and then concentrated under reduced pressure. The residue was purified by silica gel column chromatography (hexane/ethyl acetate = 1/1) to obtain 46 mg of the title compound (62% yield).
NMR(CDCl₃, 270 MHz)
δ 8.37(s, 1H), 7.39-6.90(m, 13H), 6.79(s, 1H), 4.84(dd, J=4.9,4.7 Hz, 1H), 3.94-3.73(m, 4H), 3.00(d, J=14.9 Hz, 1H), 2.58(d, J=14.9 Hz, 1H), 2.29(s, 3H), 2.22(s, 3H), 1.18(d, J=6.3 Hz, 6H), 1.11-1.04(m, 6H).

### Compound F

### (3R)-1-(2,2-bis(2-fluoroethoxy)ethyl)-3-((4-methylphenyl)aminocarbonylmethyl)-3-(N'-(4-methylphenyl)ureido)indolin-2-one

The aldehyde intermediate (50 mg) obtained above was dissolved in 2-fluoroethanol (1 mL), and then camphor sulfonic acid (5 mg) was added and the mixture was heat to reflux for 5 hours. After allowing the reaction solution to cool, toluene was added and the mixture was concentrated under reduced pressure while azeotropically removing the excess 2-fluoroethanol. The residue was diluted with dichloromethane and washed with saturated sodium bicarbonate water. The organic layer was dried (15-30°C) over anhydrous magnesium sulfate and then concentrated under reduced pressure. The residue was purified by silica gel column chromatography (hexane/ethyl acetate = 2/1) to obtain 25 mg of the title compound (41% yield).
NMR(CDCl₃, 270 MHz)
δ 7.79(s, 1H), 7.32-6.93(m, 13H), 6.76(s, 1H), 5.01-4.96(m, 1H), 4.62-4.53(m, 2H), 4.45-4.35(m, 2H), 4.11(dd, J=6.6,14.3 Hz, 1H), 4.00-3.72(m, 5H), 2.88(d, J=14.5 Hz, 1H), 2.49(d, J=14.5 Hz, 1H), 2.30(s, 3H), 2.23(s, 3H).

### Compound G

### (3R)-1-(2,2-bis(2-chloroethoxy)ethyl)-3-((4-methylphenyl)aminocarbonylmethyl)-3-(N'-(4-methylphenyl)ureido)indolin-2-one

The aldehyde intermediate (50 mg) obtained above was dissolved in 2-chloroethanol (1 mL), and then camphor sulfonic acid (5 mg) was added and the mixture was stirred at 90°C for 5 hours. After allowing the reaction solution to cool, toluene was added and the mixture was concentrated under reduced pressure while azeotropically removing the excess 2-chloroethanol. The residue was diluted with dichloromethane and washed with saturated sodium bicarbonate water. The organic layer was dried (15-30°C) over anhydrous magnesium sulfate and then concentrated under reduced pressure. The residue was purified by silica gel column chromatography (hexane/ethyl acetate = 2/1) to obtain 15 mg of the title compound (23% yield).
NMR(CDCl₃, 270 MHz)
δ 7.88(s, 1H), 7.32-6.93(m, 13H), 6.79(s, 1H), 4.96-4.91(m, 1H), 3.97-3.46(m, 10H), 2.98(d, J=14.5 Hz, 1H), 2.49(d, J=14.5 Hz, 1H), 2.30(s, 3H), 2.23(s, 3H).

### Synthesis Example 3

Compound H, the racemic form of Compound I and an optically active form of Compound I were synthesized by the following methods.

### Compound H

### (3R)-1-(2,2-diethoxyethyl)-3-(4-hydroxymethylphenyl)aminocarbonylmethyl-3-(N'-(4-methylphenyl)ureido)indolin-2-one

(+)-1- (2,2-diethoxyethyl) -3-hydroxycarbonylmethyl-3-(N'-(4-methylphenyl)ureido)indolin-2-one (compound described in "Example 174(1)" of Japanese Patent Application Laid-Open No. 7-48349, 300 mg) was dissolved in acetonitrile (10 mL), and then p-aminobenzyl alcohol (100 mg) and 1-ethyl-3-(3-dimethylaminopropyl)-carbodiimide hydrochloride (151 mg) were consecutively added and the mixture was stirred at 15-30°C for 18 hours. Water was added to the reaction solution, extraction was performed with ethyl acetate, the organic layer was dried over anhydrous sodium sulfate, and the solvent was distilled off. The obtained reaction mixture was purified by column chromatography to obtain 324 mg of the title compound (88% yield).
¹H-NMR(CDCl₃, 200 MHz)
δ 8.83(s, 1H), 7.40-6.84(m, 14H), 4.78(t, J=5.6 Hz, 1H), 4.51(s, 2H), 3.99(dd, J=5.9 Hz, 14.2 Hz, 1H), 3.83-3.45(m, 5H), 2.90(d, J=15.2 Hz, 1H), 2.67(broad s, 1H), 2.60(d, J=15.2 Hz, 1H), 1.15(t, J=6.9 Hz, 3H), 1.10(t, J=6.9 Hz, 3H).
IR(KBr, cm⁻¹)
3330, 2980, 1708, 1671, 1610, 1533, 1478, 1464, 1412, 1375, 1310, 1248, 1209, 1125, 1059, 818, 751, 475. MS(EI, 70 eV)
m/e = 560(M⁺), 514, 486, 453.

### Urea intermediate

### 3-(N'-(4-t-butyldimethylsilyloxymethylphenyl)ureido)-1-(2,2-diethoxyethyl)indolin-2-one

To a dichloromethane solution containing triphosgene (681 mg) there was slowly added dropwise a dichloromethane solution containing p-aminobenzyl-t-butyldimethylsilyl ether (1.63 g) at 15-30°C, and then a dichloromethane solution containing triethylamine (1.92 mL) was added and the mixture was stirred for one hour to prepare an isocyanate compound dichloromethane solution.

1-(2,2-diethoxyethyl)-3-(hydroxyimino)indolin-2-one (compound described as Example 28 intermediate in Japanese Patent Application Laid-Open No. 7-48349, 2.014 g) was dissolved in methanol, 10% Pd-carbon was added, and the mixture was stirred for 4 hours at 15-30°C under a hydrogen atmosphere. After filtering the reaction mixture to remove the Pd-carbon, the filtrate was concentrated. The residue was dissolved in dichloromethane and added on ice to the previously prepared isocyanate compound dichloromethane solution. After stirring for one hour at the same temperature, water was added to the reaction solution, extraction was performed with dichloromethane, and then the organic layer was dried over anhydrous sodium hydrogen sulfate and the solvent was distilled off. The reaction mixture was purified by column chromatography to obtain 2.454 g of the title compound (68% yield).
¹H-NMR(CDCl₃, 200 MHz)
δ 7.58(broad s, 1H), 7.44-6.95(m, 9H), 6.03(broad d, J=5.7 Hz, 1H), 5.21(d, J=6.6 Hz, 1H), 4.70(t, J=5.1 Hz, 1H), 4.63(s, 2H), 3.96(dd, J=5.1 Hz, 13.7 Hz, 1H), 3.83-3.42(m, 5H), 1.15(t, J=6.9 Hz, 6H), 0.94(s, 9H), 0.09(s, 6H)

### Amide intermediate

### 3-(N'-(4-t-butyldimethylsilyloxymethylphenyl)ureido)-1-(2,2-diethoxyethyl)-3-((4-methylphenyl)aminocarbonylmethyl) indolin-2-one

3-(N'-(4-t-butyldimethylsilyloxymethylphenyl)ureido)-1-(2,2-diethoxyethyl)indolin-2-one (1 g) was dissolved in dimethylformamide (15 mL), and then potassium-t-butoxide (237 mg) was added at 15-30°C, the mixture was stirred for one hour, and N-paratolyl-2-bromoacetamide (460 mg) was added. After stirring for 15 minutes, water was added, extraction was performed with ethyl acetate, the organic layer was dried over anhydrous sodium hydrogen sulfate and the solvent was distilled off. The reaction mixture was purified by silica gel column chromatography to obtain 939 mg of the title compound (73% yield) .
¹H-NMR(CDCl₃, 200 MHz)
δ 8.52(s, 1H), 7.40-6.92(m, 14H), 4.78(t, J=5.7 Hz, 1H), 4.60(s, 2H), 4.02(dd, J=5.7 Hz, 14.9 Hz, 1H), 3.87-3.46(m, 5H), 3.00(d, J=14.9 Hz, 1H), 2.67(d, J=14.9 Hz, 1H), 2.30(s, 3H), 1.17(t, J=7.4 Hz, 3H), 1.13(t, J=7.4 Hz, 3H), 0.92(s, 9H), 0.06(s, 6H).

### Compound I (racemic form)

### 1-(2,2-diethoxyethyl)-3-(N'-(4-hydroxymethylphenyl)ureido)-3-((4-methylphenyl)aminocarbonylmethyl) indolin-2-one

3-(N'-(4-t-butyldimethylsilyloxymethylphenyl)ureido)-1-(2,2-diethoxyethyl) -3-((4-methylphenyl) aminocarbonylmethyl) indolin-2-one (308 mg) was dissolved in ethanol (15 mL), concentrated sulfuric acid (10 mg) was added, and the mixture was stirred at 15-30°C for one hour. Water was added to the reaction solution, extraction was performed with ethyl acetate, the organic layer was dried over anhydrous sodium hydrogen sulfate and the solvent was distilled off. The reaction mixture was purified by silica gel column chromatography to obtain 225 mg of the title compound (88% yield).
¹H-NMR(CDCl₃, 270 MHz)
δ 8.13(s, 1H), 7.37-6.90(m, 14H), 4.79(broad s, 1H), 4.50(s, 2H), 4.01(dd, J=5.9 Hz, 14.5 Hz, 1H), 3.86-3.46(m, 6H), 2.96(d, J=14.8 Hz, 1H), 2.53(d, J=14.8 Hz, 1H), 2.29(s,3H), 1.16(t, J=7.3 Hz, 3H), 1.11(t, J=6.9 Hz, 3H).
IR (KBr, cm⁻¹)
3365, 3000, 1717, 1702, 1621, 1545, 1521, 1498, 1480, 1420, 1388, 1321, 1263, 1137, 1069, 831, 763, 515, 484.
MS (EI, 70 eV)
m/e = 560(M⁺), 515, 392, 365, 306, 292.

### Ester intermediate

### 1-(2,2-diethoxyethyl)-3-(N'-(4-t-butyldimethylsilyloxymethylphenyl)ureido)-3-((L-menthoxy)carbonylmethyl)indolin-2-one

A hexane solution containing n-butyllithium was slowly added at one equivalent (1.68 M, 1.0 mL) to a dry tetrahydrofuran solution (20 mL) containing 3-(N'-(4-t-butyldimethylsilyloxymethylphenyl)ureido)-1-(2,2-diethoxyethyl)indolin-2-one (936 mg) under a nitrogen stream while cooling on ice, and after stirring the mixture for 10 minutes at the same temperature, a dry tetrahydrofuran solution (10 mL) containing bromoacetic acid (L-menthyl) (542 mg) was added dropwise. After stirring the mixture at 0°C for 8 hours, it was poured into a saline solution and extracted with ethyl acetate. The organic layer was dried over anhydrous sodium sulfate and then concentrated, and the residue was purified by silica gel column chromatography (elution with hexane/ethyl acetate = 3/1) and recrystallized from methanol water to obtain 357 mg of the title compound (28% yield).
¹H-NMR(CDCl₃, 270 MHz)
δ 7.30-6.97(m, 8H), 6.81(brs, 1H), 6.76(brs, 1H), 4.74(dd, J=5.7 Hz, 4.9 Hz, 1H), 4.69-4.61(m, 3H), 3.94(dd, J=6.9 Hz, 14.2 Hz, 1H), 3.86-3.49(m, 5H), 2.99(d, J=15.2 Hz, 1H), 2.59(d, J=15.2 Hz, 1H), 1.92-1.90(brs, 1H), 1.21-1.10(m, 6H), 0.92(s, 9H), 1.36-0.65(m, 18H), 0.065(s, 6H).

### Compound I (optically active form)

### 1-(2,2-diethoxyethyl)-3-(N'-(4-hydroxymethylphenyl)ureido)-3-((4-methylphenyl)aminocarbonylmethyl)indolin-2-one

A potassium hydroxide aqueous solution (1N, 1.36 mL) was added to an ethanol solution (8 mL) containing 1-(2, 2-diethoxyethyl)-3-(N'-(4-t-butyldimethylsilyloxy methylphenyl)ureido)-3-((L-menthoxy)carbonylmethyl)indolin-2-one (293 mg) at 15-30°C, and the mixture was stirred at 70°C for 4 hours and then concentrated. Water was added to the residue, washing was performed with chloroform, and then 2 N hydrochloric acid was added thereto for acidity. The produced insoluble substance was extracted with ethyl acetate, and the organic layer was washed with saline solution, dried over anhydrous sodium sulfate and concentrated to obtain 234 mg of 1-(2,2-diethoxyethyl)-3-hydroxycarbonylmethyl-3-(N'-(4-hydroxymethylphenyl) ureido)indolin-2-one (carboxylic acid intermediate) as a crude product.

The carboxylic acid intermediate (234 mg) was dissolved in dichloromethane (10 mL), and then 1-ethyl-3-(3-dimethylaminopropyl)-carbodiimide hydrochloride (131 mg) and paratoluidine (73 mg) were consecutively added. The mixture was stirred at 15-30°C for 18 hours for concentration. The residue was diluted with ethyl acetate and washed with diluted hydrochloric acid and saturated sodium bicarbonate water, and then dried over anhydrous sodium sulfate for concentration. The crude product was purified by silica gel column chromatography (elution with hexane/ethyl acetate = 2/1) to obtain 204 mg of a white powder (90% ee, 80% yield from ester intermediate).

A 140 mg portion of the powder was purified by preparative high performance liquid chromatography using an optically active column to obtain the R form (117 mg, [α]_{D}²⁶=94° C=1.014/MeOH) and the S form (7 mg).

### <Preparative chromatography conditions>

Column: SUMICHIRAL OA-4800
   diameter 20 mm × length 25 cm
Detection: UV 254 nm
Flow rate: 20 mL/min.
Solvent: hexane/1,2-dichloroethane/methanol = 75/22/3
Retention times: S form: 24 min; R form: 40 min

### Example 1

Compound A as a drug with chondrogenic effect and N-methyl-2-pyrrolidone (NMP) as an organic solvent were measured out and dissolved in a proportion of 0.1 (w/w) and 1.7 (v/w), respectively, in a biodegradable L-lactic acid polymer (PLA0005, weight-average molecular weight: 5,000, product of Wako Pure Chemical Industries), to obtain a chondrogenic composition. The weight ratios of each of the components in the chondrogenic composition were PLA0005: 36.1 wt%, Compound A: 3.6 wt% and NMP: 60.3 wt%.

### Example 2

Compound A as a drug with chondrogenic effect and N-methyl-2-pyrrolidone (NMP) as an organic solvent were measured out and dissolved in a proportion of 0.1 (w/w) and 1.7 (v/w), respectively, in a biodegradable L-lactic acid/L-glycolic acid copolymer (PLGA5020, lactic acid/glycolic acid molar ratio: 50/50, weight-average molecular weight: 20,000, product of Wako Pure Chemical Industries), to obtain a chondrogenic composition. The weight ratios of each of the components in the chondrogenic composition were PLGA5020: 36.1 wt%, Compound A: 3.6 wt% and NMP: 60.3 wt%.

The chondrogenic compositions obtained in Example 1 and Example 2 were placed in an approximately 0.2 mL vessel and immersed in purified water, and then incubated at 37°C for 3 days to solidification. After lyophilizing each formulation, it was subjected to gold vapor deposition and the surface observed with a scanning electron microscope. Fig. 1 shows the results obtained using the chondrogenic composition of Example 1, and Fig. 2 shows the results obtained using the chondrogenic composition of Example 2.

As seen in the scanning electron microscope photographs of Figs. 1 and 2, the polymer precipitated (insolubilized) in the chondrogenic composition of Example 1 had pores of approximately 5-19 µm, while the polymer precipitated (insolubilized) in the chondrogenic composition of Example 2 had relatively large pores of a several dozen µm and a reticulate structure. Chondrocytes use the polymer as a scaffold for adhesion and proliferate in the pores and the reticular gaps, forming an extracellular matrix. The chondrogenic composition is gradually replaced with cartilage tissue as the polymer decomposes.

The chondrogenic composition obtained in Example 1 and the chondrogenic composition obtained in Example 2 were each measured out to approximately 10 mg in a capped tube, and a release solution (10 mM phosphate buffer containing 150 mM NaCl, 0.05% Tween80, pH 7.5) was added in an amount of 2 mL/day. The mixture was gently stirred at 37°C, after which the release solution was periodically sampled, and the content of Compound A was measured by reverse-phase HPLC. The release solution was exchanged in an amount of 2 mL/day at each sampling. The relationship between the incubation time and drug (Compound A) cumulative release rate is shown in Fig. 3. The experiment was conducted 3 times, and the mean and standard deviation (SD) are shown in the graph.

As seen from Fig. 3, the chondrogenic composition containing the biodegradable polymer with a slow decomposition rate (PLA0005) exhibited a sustained release property of approximately 3 months, while the chondrogenic composition containing the biodegradable polymer with a fast decomposition rate (PLGA5020) exhibited a sustained release property of approximately 1 month. These results demonstrate that the chondrogenic composition of the invention exhibits a decomposition rate-dependent release property.

### Reference Example 1

N-methyl-2-pyrrolidone (NMP) was added in a proportion (weight ratio) for 1.7 (v/w) to a biodegradable L-lactic acid/L-glycolic acid copolymer (PLGA5020, lactic acid/glycolic acid molar ratio: 50/50, weight-average molecular weight: 10,000, product of Wako Pure Chemical Industries) to prepare a mixture. Pulverized powder of the water-soluble compound disodium phosphate dodecahydrate was then added to and dispersed in the mixture to obtain a drug-free formulation. The weight of the disodium phosphate dodecahydrate was in a ratio of 0.2 (w/w) with respect to the weight of the PLGA5010.

### Reference Example 2

A drug-free formulation was obtained in the same manner as Reference Example 1, except that the weight of the disodium phosphate dodecahydrate pulverized was 0.3 (w/w) with respect to the PLGA5010.

### Reference Example 3

A drug-free formulation was obtained in the same manner as Reference Example 1, except that the weight of the disodium phosphate dodecahydrate pulverized powder was 0.4 (w/w) with respect to the PLGA5010.

The drug-free formulations obtained in Reference Examples 1-3 were each placed in an approximately 0.2 mL vessel, immersed in purified water and then incubated at 37°C for 3 days to solidification. After lyophilizing each formulation, it was subjected to gold vapor deposition and the surface observed with a scanning electron microscope. Figs. 4 and 5 show the results obtained using the drug-free formulation of Reference Example 1, Figs. 6 and 7 show the results obtained using the drug-free formulation of Reference Example 2, and Figs. 8 and 9 show the results obtained using the drug-free formulation of Reference Example 3.

Upon calculating the mean pore diameters of the precipitated (insolubilized) polymers based on the scanning electron microscope photographs of Figs. 4 to 9, it was found that the precipitated (insolubilized) polymer in the drug-free formulation obtained in Reference Example 1 had a mean pore diameter of 14 µm, while the precipitated (insolubilized) polymers in the drug-free formulations obtained in Reference Examples 2 and 3 had mean pore diameters of 24 µm and 29 µm, respectively. These results indicated that control of the pore size (porosity) of the precipitated (insolubilized) polymer is possible by changing the amount of water-soluble compound added.

### Example 3

Compound A as a drug with chondrogenic effect, N-methyl-2-pyrrolidone (NMP) as an organic solvent and pulverized powder of disodium phosphate dodecahydrate as a water-soluble compound were measured out and dissolved in a proportion of 0.1 (w/w), 1.7 (v/w) and 0.1 (w/w), respectively, in a biodegradable L-lactic acid/L-glycolic acid copolymer (PLGA5020, lactic acid/glycolic acid molar ratio: 50/50, weight-average molecular weight: 20,000, product of Wako Pure Chemical Industries), to obtain a chondrogenic composition. The weight ratios of each of the components in the chondrogenic composition were PLGA5020: 34.9 wt%, Compound A: 3.5 wt%, NMP: 58.1 wt% and disodium phosphate dodecahydrate: 3.5 wt%.

### Example 4

Compound A as a drug with chondrogenic effect and N-methyl-2-pyrrolidone (NMP) as an organic solvent were measured out and dissolved in a proportion of 0.1 (w/w) and 1.7 (v/w), respectively, in a biodegradable L-lactic acid/L-glycolic acid copolymer (PLGA5010, lactic acid/glycolic acid molar ratio: 50/50, weight-average molecular weight: 10,000, product of Wako Pure Chemical Industries), to obtain a chondrogenic composition. The weight ratios of each of the components in the chondrogenic composition were PLGA5010: 36.1 wt%, Compound A: 3.6 wt% and NMP: 60.3 wt%.

### Example 5

Compound A as a drug with chondrogenic effect, N-methyl-2-pyrrolidone (NMP) as an organic solvent and pulverized powder of disodium phosphate dodecahydrate as a water-soluble compound were measured out and dissolved in a proportion of 0.1 (w/w), 1.7 (v/w) and 0.1 (w/w), respectively, in a biodegradable L-lactic acid/L-glycolic acid copolymer (PLGA5010, lactic acid/glycolic acid molar ratio: 50/50, weight-average molecular weight: 10,000, product of Wako Pure Chemical Industries), to obtain a chondrogenic composition. The weight ratios of each of the components in the chondrogenic composition were PLGA5010: 34.9 wt%, Compound A: 3.5 wt%, NMP: 58.1 wt% and disodium phosphate dodecahydrate: 3.5 wt%.

### Example 6

Compound A as a drug with chondrogenic effect and N-methyl-2-pyrrolidone (NMP) as an organic solvent were measured out and dissolved in a proportion of 0.1 (w/w) and 1.7 (v/w), respectively, in a biodegradable L-lactic acid/L-glycolic acid copolymer (PLGA5005, lactic acid/glycolic acid molar ratio: 50/50, weight-average molecular weight: 5,000, product of Wako Pure Chemical Industries), to obtain a chondrogenic composition. The weight ratios of each of the components in the chondrogenic composition were PLGA5005: 36.1 wt%, Compound A: 3.6 wt% and NMP: 60.3 wt%.

### Example 7

Compound A as a drug with chondrogenic effect, N-methyl-2-pyrrolidone (NMP) as an organic solvent and pulverized powder of disodium phosphate dodecahydrate as a water-soluble compound were measured out and dissolved in a proportion of 0.1 (w/w), 1.7 (v/w) and 0.1 (w/w), respectively, in a biodegradable L-lactic acid/L-glycolic acid copolymer (PLGA5005, lactic acid/glycolic acid molar ratio: 50/50, weight-average molecular weight: 5,000, product of Wako Pure Chemical Industries), to obtain a chondrogenic composition. The weight ratios of each of the components in the chondrogenic composition were PLGA5005: 34.9 wt%, Compound A: 3.5 wt%, NMP: 58.1 wt% and disodium phosphate dodecahydrate: 3.5 wt%.

The chondrogenic compositions obtained in Examples 2-7 were each measured out to approximately 10 mg in a capped tube, and a release solution (10 mM phosphate buffer containing 150 mM NaCl, 0.05% Tween80, pH 7.5) was added in an amount of 2 mL/day. The mixture was gently stirred at 37°C, after which the release solution was periodically sampled and the content of Compound A was measured by reverse-phase HPLC. The release solution was exchanged in an amount of 2 mL/day at each sampling. The relationship between the incubation times and drug (Compound A) cumulative release rates is shown in Figs. 10 and 11. The experiment was conducted 3 times, and the mean and standard deviation (SD) are shown in the graph.

Fig. 10 shows the results of using the chondrogenic compositions of Examples 2, 4 and 6 which contained no disodium phosphate dodecahydrate as a water-soluble compound, and Fig. 11 shows the results of using the chondrogenic compositions of Examples 3, 5 and 7 which contained disodium phosphate dodecahydrate as a water-soluble compound.

As seen in Figs. 10 and 11, the drug release rates (especially the initial drug release rates up to day 21 of incubation) were dependent on the molecular weight, both with and without the disodium phosphate dodecahydrate pulverized powder, with the formulations comprising polymers of greater molecular weight giving lower release rates.

As will also be understood by comparing Figs. 10 and 11, addition of disodium phosphate dodecahydrate pulverized powder increased the drug release rate of the formulation (especially the initial drug release rate up to day 21 of incubation). This tendency was independent of the difference in molecular weight of the polymer. The results demonstrate that the drug release rate of a chondrogenic composition according to the invention can be controlled by the molecular weight of the polymer and the content of the water-soluble compound added.

The drug balance of each of the chondrogenic compositions obtained in Examples 2-7 at the end of the release experiment (incubation time: 63 days) was also investigated. The drug balance is the sum of the drug cumulative release rate and the drug residue rate in the formulation. The results for the drug balance are shown in Figs. 12 and 13. Fig. 12 shows the results of using the chondrogenic compositions of Examples 2, 4 and 6 which contained no disodium phosphate dodecahydrate as a water-soluble compound, and Fig. 13 shows the results of using the chondrogenic compositions of Examples 3, 5 and 7 which contained disodium phosphate dodecahydrate as a water-soluble compound.

As will be understood by comparing Figs. 12 and 13, the drug balance for the formulations containing disodium phosphate dodecahydrate pulverized powder was greater than the drug balance for the formulations which did not contain it. This tendency was independent of the molecular weight of the polymer used. The improvement in drug balance was attributed to the fact that pH reduction produced by decomposition of the polymer was neutralized by the basic water-soluble compound, and to the fact that pH reduction was inhibited by rapid release of produced acidic substances from the composition.

These results demonstrated that addition of a basic compound such as disodium phosphate dodecahydrate to the chondrogenic composition inhibits pH reduction in the formulation during the drug release period, thus stabilizing the drug. This pH inhibition is believed to also contribute to suppressed irritation in the body.

### Example 8

In the same manner as Example 1, Compound A as a drug with chondrogenic effect and N-methyl-2-pyrrolidone (NMP) as an organic solvent were measured out and dissolved in a proportion of 0.1 (w/w) and 1.7 (v/w), respectively, in a biodegradable L-lactic acid polymer (PLA0005, weight-average molecular weight: 5,000, product of Wako Pure Chemical Industries), to obtain a chondrogenic composition. The weight ratios of each of the components in the chondrogenic composition were PLA0005: 36.1 wt%, Compound A: 3.6 wt% and NMP: 60.3 wt%.

A 2.4 mm diameter Kirschner wire (Mizuho Medical Instruments) was used to create a defect in the right femoral patellar groove surface of ten-week-old male SD rats (Clea Japan, Inc.), to a depth of 2.5 mm reaching the bone marrow. Immediately afterward, approximately 10 µL of the aforementioned chondrogenic composition was dropped thereon, after which physiological saline (Otsuka Pharmaceutical Co., Ltd.) was dropped thereover for solidification. The incision was sutured, and the rats were raised for 10 weeks after surgery and then autopsied for visual observation of the effect on cartilage repair at the site of damage.

The defect-created femurs were isolated, immersed in 20% neutral-buffered formalin and fixed at room temperature for one week. After completion of fixing, they were demineralized for 2 weeks in an aqueous solution of 20% EDTA-4Na (pH 7.4, product of Wako Pure Chemical Industries), and the defected sites were trimmed with a razor (Feather Safety Razor Co., Ltd.). The trimmed sites were prepared into paraffin blocks using an automatic embedder (Sakura Finetechnical Co., Ltd.) and paraffin (equal mixture of products by Kokusan Chemical Co., Ltd. and Fisher Scientific Co.). An automatic paraffin dispensing/embedding center (Miles Scientific Co., Ltd.) was used to fix the paraffin blocks to a tissue sample cassette (TISSUE-TEK Co., Ltd.). The paraffin blocks were cut into 2 µm-thick sections using a microtome (Yamato Kohki Industrial Co., Ltd.) and a microtome knife (Feather Safety Razor Co., Ltd.), and then attached to slide glass (Matsunami Glass Co., Ltd.) and dried.

The sections were dipped in xylene (Wako Pure Chemical Industries) to remove the paraffin, and then dipped in a gradual dilution series from ethanol to water, and stored in water. The sections were dyed by immersion in 0.3% Safranine O aqueous solution (Merck Japan Ltd.), and after washing and then dehydration with a graded ethanol series (Wako Pure Chemical Industries), they were penetrated with xylene (Wako Pure Chemical Industries), and mounted with mounting medium (Sakura Finetechnical Co., Ltd.) and a cover glass (Matsunami Glass Co., Ltd.) to prepare Safranine. O dyed specimens. The specimens were histologically observed using an optical microscope (Nikon Corporation, 10x magnification (objective lens)). The tests were conducted on 5 individuals for each example.

### Comparative Example 1

N-methyl-2-pyrrolidone (NMP) as an organic solvent was measured out and dissolved in a proportion of 1.7 (v/w) in a biodegradable L-lactic acid polymer (PLA0005, weight-average molecular weight: 5,000, product of Wako Pure Chemical Industries) to obtain a drug-free formulation. The weight ratios of each of the components in the obtained drug-free formulation were PLA0005: 37.5 wt% and NMP: 62.5 wt%.

The results of observation of 5 individuals in Example 8 and Comparative Example 1 are summarized below in Table 10.

**Table 10**

| Formulation | Individual No. | Macroscopic observation | Histological observation |
|---|---|---|---|
| | | Condition of defected site (*1) | Repair by hyaline cartilage (*2) |
| Formulation containing no Compound A | 1 | depressed | - |
| | 2 | depressed | - |
| | 3 | depressed | - |
| | 4 | depressed | +/- |
| | 5 | depressed | - |
| Formulation containing 10% Compound A | 6 | smooth | + |
| | 7 | depressed | - |
| | 8 | smooth | +++ |
| | 9 | depressed | - |
| | 10 | smooth | ++ |

| | | | |
|---|---|---|---|
| (*1) Condition of damaged site: Indicates relationship between defected site and normal surrounding articular cartilage. Depressed = Concavity at defected site. Smooth = Repaired tissue at defected site, filled in to height of normal articular cartilage. | | | |
| (*2) Repair by hyaline cartilage: - = Not found, +/- = Slight, + = Mild, ++ = Moderate, +++ = Marked | | | |

As seen from Table 10, the drug-free formulation containing no Compound A had insufficient replacement of the defected site by repair tissue, and the defected sites were depressed in all of the individuals. Histologically, only one of the five exhibited formation of hyaline cartilage tissue, while absolutely no hyaline cartilage was found in the other individuals. On the other hand, the chondrogenic composition of the invention which contained Compound A induced greater replacement of the defected sites by repair tissue than the drug-free formulation, with three of the five individuals exhibiting no depression at the defected sites and confluency with the surrounding normal articular cartilage. Histologically, distinct cartilage repair by hyaline cartilage tissue was apparent in three of the individuals. These results indicated that a chondrogenic composition containing Compound A according to the invention exhibits a satisfactory cartilage repair effect for articular cartilage damage.

### Industrial Applicability

As explained above, the present invention provides a chondrogenic composition which does not require a high level of skill or sophisticated equipment for treatment, and which offers excellent biocompatibility as well as adequately high efficiency for repair and regeneration of cartilage.

## Claims

1. A chondrogenic composition comprising a drug with chondrogenic effect, a biodegradable and/or bioerodable polymer and a water-soluble and/or water-dispersible organic solvent.

2. A chondrogenic composition according to claim 1, wherein said drug with chondrogenic effect is a compound represented by the following general formula (I) or a salt thereof. 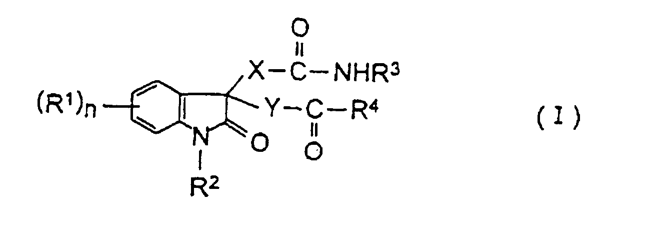 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent;
R³ represents a lower alkyl group with an optional substituent, a cycloalkyl group with an optional substituent, an aryl group with an optional substituent or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group;
X and Y may be the same or different and each represents -CH₂-, -NH- or -O-, and n represents an integer of 0-4.

3. A chondrogenic composition according to claim 2, wherein R¹ is a halogen atom, a lower alkyl group, a lower alkoxy group or a nitro group; R² is a hydrogen atom, a lower alkyl group with an optional substituent, a lower alkenyl group with an optional substituent or an aryl group with an optional substituent; R³ is a lower alkyl group with an optional substituent, a cycloalkyl group with an optional substituent or an aryl group with an optional substituent; R⁴ is a lower alkyl group with an optional substituent, an aryl group with an optional substituent, a heterocyclic group with an optional substituent, -OR⁵ or -NR⁶R⁷ wherein R⁵, R⁶ and R⁷ are as previously defined; X is -CH₂-, -NH- or -O-; Y is -CH₂- or -NH-; and n is 0 or 1.

4. A chondrogenic composition according to claim 2, wherein R² is a lower alkyl group with an optional substituent which is optionally substituted with a halogen atom; R³ is an aryl group with an optional substituent; R⁴ is -NR⁶R⁷ wherein R⁶ and R⁷ may be the same or different and each represents a hydrogen atom or an aryl group with an optional substituent; X and Y may be the same or different and each represents -CH₂ or -NH-; and n is 0.

5. A chondrogenic composition according to claim 4, wherein R² is a lower alkyl group substituted at the same carbon with two lower alkoxy groups which are optionally substituted with 1-5 halogen atoms or the group -O-Z-O- wherein Z represents a lower alkylene group optionally substituted with 1-10 halogen atoms; R³ is an aryl group which has a lower alkyl group or an amino group which amino group optionally has a lower alkyl group; and R⁴ is -NR⁶R⁷ wherein R⁶ and R⁷ may be the same or different and each represents a hydrogen atom or an aryl group which has a lower alkyl group or an amino group which amino group optionally has a lower alkyl group.

6. A chondrogenic composition according to claim 5, wherein R² is a group represented by the following general formula (II): 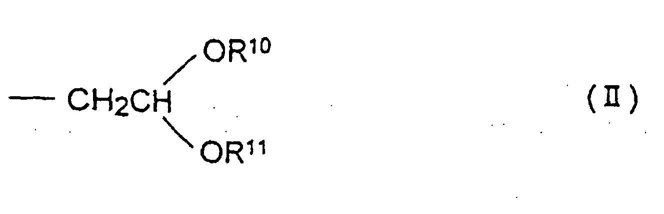 wherein R¹⁰ and R¹¹ may be the same or different, and each represents a lower alkyl group optionally substituted with 1-5 halogen atoms,
or general formula (III) : 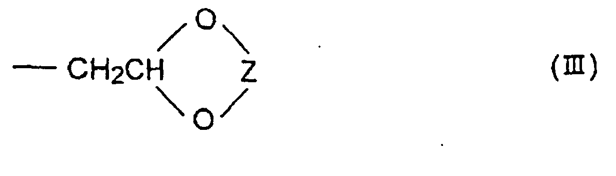 wherein Z represents a lower alkylene group optionally substituted with 1-10 halogen atoms.

7. A chondrogenic composition according to claim 6, wherein either or both R¹⁰ and R¹¹ are lower alkyl groups with 1-5 halogen atoms.

8. A chondrogenic composition according to claim 6, wherein R² is a 2,2-diethoxyethyl group, a 2,2-dimethoxyethyl group, a 2,2-diisopropoxyethyl group, a 2,2-bis(2-fluoroethoxy)ethyl group or a 2,2-bis(2-chloroethoxy)ethyl group, X is -NH- and Y is -CH₂-.

9. A chondrogenic composition according to claim 6, wherein R³ is a 4-methylphenyl group, X is -NH- and Y is -CH₂-.

10. A chondrogenic composition according to claim 6, wherein R⁴ is -NHR⁷ wherein R⁷ is a 4-methylphenyl group, a 4-ethylphenyl group or a 4-(N,N-dimethylamino)phenyl group, X is -NH- and Y is -CH₂-.

11. A chondrogenic composition according to claim 6, wherein the combination of R², R³ and R⁴ is any of the following:
R² is a 2,2-diethoxyethyl group, R³ is a 4-methylphenyl group and R⁴ is -NHR⁷ wherein R⁷ is a 4-methylphenyl group;
R² is a 2,2-diethoxyethyl group, R³ is a 4-methylphenyl group and R⁴ is -NHR⁷ wherein R⁷ is a 4-ethylphenyl group;
R² is a 2,2-diethoxyethyl group, R³ is a 4-methylphenyl group and R⁴ is -NHR⁷ wherein R⁷ is a 4-(N,N-dimethylamino)phenyl group;
R² is a 2,2-dimethoxyethyl group, R³ is a 4-methylphenyl group and R⁴ is -NHR⁷ wherein R⁷ is a 4-methylphenyl group;
R² is a 2,2-diisopropoxyethyl group, R³ is a 4-methylphenyl group and R⁴ is -NHR⁷ wherein R⁷ is a 4-methylphenyl group;
R² is a 2,2-bis(2-fluoroethoxy)ethyl group, R³ is a 4-methylphenyl group and R⁴ is -NHR⁷ wherein R⁷ is a 4-methylphenyl group; or
R² is a 2,2-bis (2-chloroethoxy) ethyl group, R³ is a 4-methylphenyl group and R⁴ is -NHR⁷ wherein R⁷ is a 4-methylphenyl group.

12. A chondrogenic composition according to claim 4, wherein R² is a 2,2-diethoxyethyl group, R³ is a 4-methylphenyl group and R⁴ is -NHR⁷ wherein R⁷ is a 4-methylphenyl group.

13. A chondrogenic composition according to claim 1, wherein said drug with chondrogenic effect is a compound represented by the following general formula (IV) or a salt thereof. 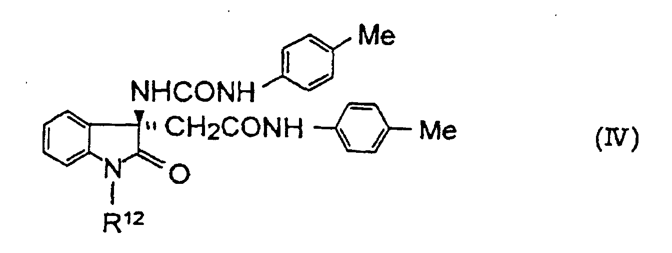 wherein R¹² represents a lower alkyl group substituted at the same carbon with two lower alkoxy groups which is substituted with 1-5 halogen atoms.

14. A chondrogenic composition according to claim 13, wherein R¹² is a group represented by general formula (V) : 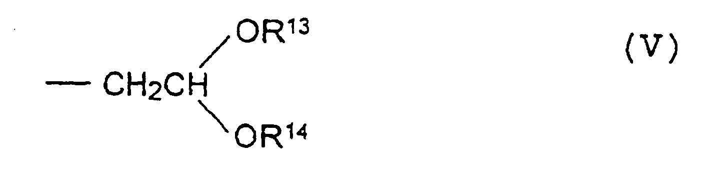 wherein R¹³ and R¹⁴ may be the same or different, and each represents a lower alkyl group substituted with 1-5 halogen atoms.

15. A chondrogenic composition according to claim 14, wherein R¹³ and R¹⁴ may be the same or different, and each represents an ethyl group substituted with a halogen atom.

16. A chondrogenic composition according to claim 14, wherein R¹² is a 2,2-bis(2-fluoroethoxy)ethyl group or a 2,2-bis(2-chloroethoxy)ethyl group.

17. A chondrogenic composition according to claim 1, wherein said polymer is a porous body-forming polymer which holds proliferated chondrocytes induced by said drug with chondrogenic effect in the body.

18. A chondrogenic composition according to claim 1, wherein said polymer has a weight-average molecular weight of 500 or greater.

19. A chondrogenic composition according to claim 1, wherein said polymer is at least one polymer selected from the group consisting of polyhydroxyalkanoic acid, lactone ring-opened polymer, hydroxyalkanoic acid/lactone copolymer, polyacetal, polyketal, polymethylvinyl ether, chitin, chitosan, polyamide, polyamino acid, polyurethane, polyester amide, polycarboxylic acid, polyanhydride, polyalkylene, polyphosphoric acid, polyortho ester and a copolymer containing at least one of these in the molecule.

20. A chondrogenic composition according to claim 19, wherein said polymer is a polymer or copolymer of a molecule selected from the group consisting of glycolic acid, lactic acid, hydroxybutyric acid and hydroxyvaleric acid.

21. A chondrogenic composition according to claim 1, wherein at least a portion of said polymer is a reactive polymer having an unsaturated double bond.

22. A chondrogenic composition according to claim 21, wherein said reactive polymer is a compound having a skeleton comprising at least one polymer selected from the group consisting of polyhydroxyalkanoic acid, lactone ring-opened polymer, hydroxyalkanoic acid/lactone copolymer, polyacetal, polyketal, polymethylvinyl ether, chitin, chitosan, polyamide, polyamino acid, polyurethane, polyester amide, polycarboxylic acid, polyanhydride, polyalkylene, polyphosphoric acid, polyortho ester and a copolymer containing at least one of these in the molecule, with at least one (meth)acryloyl group bonded to the skeleton.

23. A chondrogenic composition according to claim 1, wherein said organic solvent is at least one organic solvent selected from the group consisting of N-methyl-2-pyrrolidone, 2-pyrrolidone, ethanol, benzyl alcohol, propyleneglycol, acetone, ethyl acetate, methyl ethyl ketone, dimethylformamide, dimethylsulfoxide, tetrahydrofuran, caprolactam, N,N-dimethylacetamide, triacetin, glycerol, polyoxyethylene-hardened castor oil, vitelline phospholipid, soy lecithin, polyoxyethylene sorbitan fatty ester and polyoxyethylene alkylether.

24. A chondrogenic composition according to claim 23, wherein said organic solvent is N-methyl-2-pyrrolidone.

25. A chondrogenic composition according to claim 1, which further comprises a water-soluble compound.

26. A chondrogenic composition according to claim 25, wherein said water-soluble compound has a mean particle size of 1-3000 µm.

27. A chondrogenic composition according to claim 25, wherein said water-soluble compound is at least one water-soluble compound selected from the group consisting of monosodium phosphate, monosodium phosphate hydrate, disodium phosphate, disodium phosphate hydrate, sodium chloride, magnesium sulfate, magnesium sulfate hydrate, magnesium sulfate double salt, magnesium hydroxide, glucose, mannitol, xylose, sucrose, fructose, hyaluronic acid, dextran, amino acid and polypeptide.

28. A chondrogenic composition according to claim 25, wherein said water-soluble compound is a compound that produces a pH of 6 or greater in aqueous solution.

29. A chondrogenic composition according to claim 1, wherein the content of said drug with chondrogenic effect is 0.0001-50 wt%, the content of said polymer is 5-80 wt% and the content of said organic solvent is 5-90 wt%.

30. A chondrogenic composition according to claim 25, wherein the content of said water-soluble compound is no greater than 90 wt%.
